(19)

Europäisches
Patentamt
European
Patent Office
Office européen
des brevets

(11) **EP 2 747 028 B1**

(12) **EUROPEAN PATENT SPECIFICATION**

(45) Date of publication and mention
of the grant of the patent:
**19.08.2015  Bulletin 2015/34**

(51) Int Cl.:
***G06T 7/00*** *(2006.01)*

(21) Application number: **12382512.7**

(22) Date of filing: **18.12.2012**

(54) **Method for recovering a relative depth map from a single image or a sequence of still images**

Verfahren zum Erstellen einer relativen Tiefenkarte von einem einzelnen Bild oder einer Sequenz
stehender Bilder

Procédé de récupération d'une carte de profondeur relative à partir d'une image unique ou d'une
séquence d'images fixes

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(43) Date of publication of application:
**25.06.2014  Bulletin 2014/26**

(73) Proprietor: **Universitat Pompeu Fabra
08002 Barcelona (ES)**

(72) Inventors:
• **Caselles Costa, Vicent
08015 Barcelona (ES)**
• **Calderero Patino, Felipe
08029 Barcelona (ES)**

(74) Representative: **Carvajal y Urquijo, Isabel et al
Clarke, Modet & Co.
Suero de Quiñones, 34-36
28002 Madrid (ES)**

(56) References cited:
• YU S X ET AL: "Inferring spatial layout from a
single image via depth-ordered grouping",
COMPUTER VISION AND PATTERN
RECOGNITION WORKSHOPS, 2008. CVPR
WORKSHOPS 2008. IEEE COMPUTER SOCIETY
CONFERENCE ON, IEEE, PISCATAWAY, NJ,
USA, 23 June 2008 (2008-06-23), pages 1-7,
XP031285533, ISBN: 978-1-4244-2339-2
• NA-EUN YANG ET AL: "Depth map generation
from a single image using local depth
hypothesis", CONSUMER ELECTRONICS (ICCE),
2012 IEEE INTERNATIONAL CONFERENCE ON,
IEEE, 13 January 2012 (2012-01-13), pages
311-312, XP032124907, DOI: 10.1109/ICCE.
2012.6161883 ISBN: 978-1-4577-0230-3
• FELIPE CALDERERO ET AL: "Recovering
Relative Depth from Low-Level Features Without
Explicit T-junction Detection and Interpretation",
INTERNATIONAL JOURNAL OF COMPUTER
VISION, 7 February 2013 (2013-02-07),
XP055062749, ISSN: 0920-5691, DOI:
10.1007/s11263-013-0613-4

## Description

*FIELD OF THE INVENTION*

The present invention is comprised within the field of monocular depth estimation, by which a depth map with relative depth information is extracted from a single image or a sequence of still images.

*BACKGROUND OF THE INVENTION*

The human visual system is able to perceive depth even from a single and still image thanks to monocular (static) depth cues [25]. These cues are also known as pictorial cues, since they have been used by painters for centuries in their search for realistic effects in their masterpieces.

In the context of computer vision, the monocular depth estimation problem can be defined as inferring the depth order of the objects present in a scene using only information from a single view or image. Except in cases where the size of the objects is known a priori, in general, the extracted depth relations are relative, meaning that it can only be concluded that an object is closer or farther (or at the same depth) to the camera than another object, but no information about its absolute depth position can be extracted.

Monocular depth cues have been extensively studied by psychologist during the last century, using psycho-visual and perceptual studies [28]. The use of monocular cues for depth estimation has been profoundly analyzed in the scientific literature. These cues can be mainly classified in three types: high-level, mid-level, and low-level cues.

High-level cues do involve image understanding from a semantic point of view, and usually require previous object extraction and recognition stages to be inferred. Some of the most relevant are relative and known size, or light and shadow distribution.

Mid-level cues as those that require a basic knowledge of the image content, such as image structure [57]. Some examples are perspective, that requires detecting first the presence of projective distortions of the scene; texture gradient, based on previous extraction of textured areas of the image; or atmospheric perspective, that can be only applied in landscapes or large depth range images.

On the contrary, low-level cues do not necessarily require a high level analysis of the image (although they can benefit from it) and can be directly and locally inferred. Among them, let us mention blur [42], convexity, closure, and intersection (also known as occlusion) cues. Blur appears in objects outside the depth of field of the camera (the distance between the nearest and farthest objects in a scene that appear acceptably sharp). It has been shown to be a valuable cue for monocular depth estimation [42]. When all imaged objects are inside the depth of field of the camera (that is, focused), blur information cannot help to estimate depth relations and it is necessary to rely on other cues such as convexity, closure, and occlusion.

Occlusion cues can be used to compute low-level ordinal depth information for images in the depth of field. Surprisingly, they have comparatively received little attention in the image processing and computer vision literatures and the problem of occlusion based relative depth estimation can be still considered as an open one. On the contrary, a great effort has been invested to develop algorithms based on cues that require multiple images, such as depth from stereo [35], depth from motion [18], or depth from defocus [17].

Occlusion-based monocular depth estimation is a fundamental problem in psycho-physics. In fact, psycho-visual studies suggest that this type of local information is extracted and used in early vision stages to rapidly complete partially-occluded objects [51], being a key step for the next stages of the human visual system. Interpreted from the image processing perspective this means that semantic image analysis and high level applications would significantly benefit from local depth image relationships, in terms of robustness, accuracy, simplicity, and efficiency.

Computer vision approaches dealing with occlusion-based monocular depth estimation have been deeply influenced by psycho-visual ideas of the Gestalt psychology school [41], and particularly, by the work of Gaetano Kanizsa [29], that describes T-junctions (and convexity) as fundamental atoms of monocular depth perception. For that reason, most approaches in this area focus on developing computational and mathematical attempts to model, extract, and interpret T-junctions in digital images. In agreement with the previous discussion, the benefits of considering T-junction local depth information at early stages have been demonstrated by [9], where an invariant scale-space filter preserving T-junctions significantly simplifies the image while preserving all relevant visual information.

The family of monocular depth estimation techniques based on T-junction cues (from now on, referred to as "Gestalt-based techniques") was initiated by the inspiring and pioneering work of Nitzberg and Mumford [43] (see also [44]), which proposes a joint segmentation and depth extraction model to compute a 2.1D sketch of the image. The 2.1D sketch [40] is a low-level depth-based image representation, where regions of the image are assumed to form planes (layers) whose normals lie along the camera viewing direction. Using a variational approach, Nitzberg et al. [43] define an energy functional in terms of region simplification and completion of occluded contours by linking T-junctions. Despite its theoretical importance, the combinatorial complexity arisen in this energy minimization problem makes the approach far from practical for real images.

One way to reduce the computational demands of the original problem is to consider segmentation and depth estimation as two independent problems. For instance, the image can be segmented using color information (without considering depth) and, then, T-junctions are extracted and analyzed from the simplified image partition. This is the strategy followed by [3] in order to build a global depth ordering in the form of a 2.1D sketch. After segmenting the image, depth estimation is formulated as a quadratic optimization problem in terms of the T-junction interpretation. The algorithm relies on the assumption that a perfect object extraction is provided by color image segmentation. For generic images, this is a strong (and unrealistic) assumption, since image segmentation is a difficult and open problem itself. In that sense, the challenge is moved from depth interpretation to image segmentation. Additionally, local depth relations are not used as part of the fundamental low-level information, since they are not considered at all to compute the image segmentation.

A similar strategy has been followed in [12] and [23] where, given a previous image segmentation, the authors formulate depth estimation as a layer image representation problem [60]. For that purpose, they solve an optimization problem formulated, respectively, in a Markov Random Field (MRF) and a Minimum Description Length (MDL) framework. Both approaches rely on the quality of the initial segmentation and, in some cases, they introduce heuristic assumptions about the number of scene layers.

A common criticism to the previous approaches is that they require hard decisions based on optimization strategies. On one hand, this makes most of the approaches sensitive to detection problems and combinatorially complex. On the other, they become difficult to apply to real images where a large number of T-junctions exist. This fact explains that, in most cases, the depth ordering is provided for either simple images, or for highly simplified (error-free) image segmentations. In this context, not only objective but also visual evaluation or comparison between techniques becomes impossible.

To avoid considering segmentation and depth estimation as two completely independent processes, the authors of [14,15] compute a color-based image segmentation constrained by local depth information inferred from T-junctions. First, they incorporate ordering information provided by T-junctions into a region merging algorithm [8]. The regions belonging to a T-junction candidate are not allowed to merge, which prevents the destruction of valuable depth information in the final partition. Finally, the depth relations of the partition are studied and modified using a graph model, to provide a globally depth-coherent image representation.

Following a similar methodology, the approach in [45] goes one step further and explicitly considers T-junction information as part of the merging cost used to create the image segmentation. During the region merging process, local depth information (involving contrast, angle, and curvature cues) increases the merging cost of regions that do not belong to the same depth plane. This is done through a term based on the probability of observing a T-junction formed by the two regions under consideration and a common neighbor region. Finally, a pruning strategy is applied to solve depth conflicts, converging to an unambiguous relative depth order.

From a different perspective, the work in [13] proposes a phenomenological approach based on extracting a set of low- and mid-level cues, such as T-junctions, X-junctions, local convexity (curvature sign), and visual completion (segment alignment). Straight line segments are extracted using a robust edge detector [59], and T-and X-junction candidates are reconstructed using good continuation principles. Pixels holding the local depth cues receive a positive value and the rest are set to zero. This generates local hard decisions about the relative order of the pixels that, in turn, are globally integrated using a non-linear diffusion strategy guided by the image color information. The diffusion process is implemented using Yaroslavsky's neighborhood filter [63] in an iterative manner.

Gestalt-based approaches propose a formal model inherited from Gestalt psychology. There is no warranty that a good psycho-visual model, that succeeds to explain perception mechanisms, would also provide a suitable mathematical framework from the image processing viewpoint. The lack of proper modeling and formalization may have also contributed to increase the difficulty of the monocular depth estimation problem. Most approaches in the literature suffer from this "original sin" and, in fact, most of them are based on two stages: T-junction extraction (which is a difficult problem itself for discrete images [46]) and T-junction interpretation (usually by minimizing some cost function). This methodology leads not to low-level but to mid-level depth cues since they rely on previous estimation stages, such as edge detection and edge completion.

The other family of monocular depth estimation techniques can be described as the supervised learning based approaches. In this case, the system is previously trained against a large set of ground truth data (for instance, manually labeled examples or real depth maps). Relevant features in terms of color, texture, shape descriptors, and orientation features may be extracted from the training stage and used to interpret new examples.

For instance, in [53] the image is previously over-segmented and, then, color, texture, and shape features are extracted at different scales for each region of the segmentation. Depth information is inferred using a discriminatively-trained Markov Random Field (MRF) that incorporates multi-scale local and global image features, and models both depths at individual points as well as the relation between depths at different points.

A similar approach is proposed in [27], where the authors consider an image over-segmentation computed by a watershed transform to extract region features. Then, weak boundaries between regions, according to the occlusion confidence, are sequentially removed until the weakest boundary has a confidence greater than some threshold. The strength of the boundaries is inferred from the ground truth data using a Conditional Random Field (CRF).

The approach presented in [38] performs first a semantic classification of image pixels in a set of labels (sky, road, water, grass, tree, building, mountain and foreground object), using a multi-class image labeling method that produces pixel-level semantic annotations. Based on semantic classes of pixels or regions, depth and geometry constraints are enforced, including several camera and picture constraints, such as the focal length or the vertical axis absolute position. Ground truth data is used to model prior distributions for object classes, and to infer depth by measuring the change in appearance within a given semantic class in terms of absolute depth position. Under these assumptions and for specific image types, the results provide absolute depth values.

Finally, the monocular depth estimation problem can be considered as a generalization of a simpler problem known as the figure/ground assignment problem [52]. In the context of computer vision, figure/ground organization (also referred as boundary ownership problem) amounts to assign the pixels of the image to figure (front) or background (back) classes. In general, this step is not directly performed on image pixels but on a previously computed image segmentation or boundary map. Several approaches have been proposed in the literature, such as [19, 36, 39]. For instance, in [39] a joint image segmentation and figure/ground organization is presented. Both processes are coupled in a common image representation that encodes affinity and ordering preferences and that results from solving an Angular Embedding problem [64].

Learning-based approaches avoid the complexity of modeling depth perception mechanisms in discrete images, at the cost of extensive training. Hence, they are dependent on the availability of specific ground truth data and their use is limited to trained image types, which hinds their generalization to other image classes, or when there is no prior knowledge of the image content. Although they do not require an explicit depth model, it is still necessary to define reliable features to drive the training and classification stages. Despite the previous drawbacks, they provide accurate results in terms of relative, and even absolute depth, when they are applied on the kind of (real) images they were trained for.

The monocular depth estimation problem has also been widely discussed in patent literature. Some methodologies extract depth maps using additional information not contained in the image itself. For instance, patent document WO2011138472-A1 discloses generation of depth maps from a series of still images by using optical flow analysis, whereas in document WO2008016885-A2 autofocusing process information is employed.

Other methodologies disclosed in patent literature allow extracting depth order information from a single and still image:

- Segmentation is practiced in many of patent documents, such as in document US20100079448-A, where image is divided into segments with similar characteristics (colour, intensity), or in document WO2006003577-A1, where image is segmented in foreground and background.
- Edge detection is disclosed in documents EP2169619-A2 and US2010079453-A1, the latter also employing line detection and vanishing point detection.
- Detection and interpretation of T-junctions is disclosed in documents WO2005052858-A1 and WO2005015497-A1.
- Classification of the image is carried out in document US20110188773-A1, where image is first classified in different categories (landscape image, indoor image, city image), prior to depth map generation.
- Learning techniques are disclosed in document US20120002871-A1.
- Overlapping techniques are disclosed in US20120293499-A1, where it is required to divide first the image into regions.
- Luminance and chrominance analysis of the image are performed in documents US2006061569-A1 and US20080247670-A1, respectively.
- Image blur is studied in documents US20070024614-A1, US2010310176-A1 y US2010080481-A1. In document CN102509294-A a blur image is obtained from the original image to estimate the depth map.
- Low-level techniques are disclosed in document WO10018880-A1, where different sizes of windows are applied to the image to first generate local deviation images and later to create equalized images from which a depth map can be estimated, and in document US20070146232-A1, where for each pixel of the image depth values are assigned based on a cost function.

In conclusion, learning-based techniques are a valuable solution to estimate monocular depth of specific image classes when ground truth data is available. In more general situations, Gestalt-based techniques are more appropriate. Nevertheless, in both cases, there is a lack of low-level modeling and formalization of the monocular depth features that contribute to the depth perception: learning-based approaches skip this stage, and Gestalt-based approaches use a mid-level T-junction model inherited from psychology.

*References*

1. Alvarez, L., Gousseau, Y., Morel, J.: Scales in natural images and a consequence on their bounded variation norm. Scale-Space Theories in Computer Vision pp. 247-258 (1999)

2. Alvarez, L., Gousseau, Y., Morel, J.: The size of objects in natural and artificial images. Advances in Imaging and Electron Physics 111, 167-242 (1999)

3. Amer, M., Raich, R., Todorovic, S.:Monocular extraction of 2.1 d sketch. In: Proc. of the International Conference on Image Processing (2010)

4. Arbelaez, P., Maire, M., Fowlkes, C., Malik, J.: Contour detection and hierarchical image segmentation. IEEE Trans. Pattern Anal. Mach. Intell. 33(5), 898-916 (2011)

5. Bordenave, C., Gousseau, Y., Roue , F.: The dead leaves model: a general tessellation modeling occlusion. Advances in Applied Probability 38(1), 31-46 (2006)

6. Buades, A., Coll, B., Morel, J.: A non-local algorithm for image denoising. In: Computer Vision and Pattern Recognition, 2005. CVPR 2005. IEEE Computer Society Conference on, vol. 2, pp. 60-65. Ieee (2005)

7. Buades, A., Le, T., Morel, J., Vese, L.: Fast cartoon+ texture image filters. Image Processing, IEEE Transactions on 19(8), 1978-1986 (2010)

8. Calderero, F., Marques, F.: Region merging techniques using information theory statistical measures. Image Processing, IEEE Transactions on 19(6), 1567-1586 (2010)

9. Caselles, V., Coll, B., Morel, J.: A kanizsa programme. ICAOS'96 pp. 356-359 (1996)

10. Caselles, V., Coll, B., Morel, J.: Topographic maps and local contrast changes in natural images. International Journal of Computer Vision 33(1), 5{27 (1999)

11. Caselles, V., Monasse, P.: Geometric description of images as topographic maps, vol. 1984. Springer-Verlag New York Inc (2010)

12. Darrell, T., Pentland, A.: Cooperative robust estimation using layers of support. Pattern Analysis and Machine Intelligence, IEEE Transactions on 17(5), 474-487 (1995)

13. Dimiccoli, M., Morel, J., Salembier, P.: Monocular depth by nonlinear di usion. In: Computer Vision, Graphics & Image Processing, 2008. ICVGIP'08. Sixth Indian Conference on, pp. 95-102. IEEE (2008)

14. Dimiccoli, M., Salembier, P.: Exploiting t-junctions for depth segregation in single images. In: Acoustics, Speech and Signal Processing, 2009. ICASSP 2009. IEEE International Conference on, pp. 1229-1232. IEEE (2009)

15. Dimiccoli, M., Salembier, P.: Hierarchical region-based representation for segmentation and filtering with depth in single images. In: Image Processing (ICIP), 2009 16th IEEE International Conference on, pp. 3533-3536. IEEE (2009)

16. Eisemann, E., Durand, F.: Flash photography enhancement via intrinsic relighting. In: ACM Transactions on Graphics (TOG), vol. 23, pp. 673-678. ACM (2004)

17. Favaro, P., Soatto, S., Burger, M., Osher, S.: Shape from defocus via di usion. Pattern Analysis and Machine Intelligence, IEEE Transactions on 30(3), 518-531 (2008)

18. Feldman, D., Weinshall, D.: Motion segmentation and depth ordering using an occlusion detector. Pattern Analysis and Machine Intelligence, IEEE Transactions on 30(7), 1171-1185 (2008)

19. Fowlkes, C., Martin, D., Malik, J.: Local figure-ground cues are valid for natural images. Journal of Vision 7(8) (2007)

20. Froyen, V., Feldman, J., Singh, M.: A bayesian framework for figure-ground interpretation. Advances in Neural Information Processing Systems 23 (2010)

21. Froyen, V., Feldman, J., Singh, M.: Local propagation of border-ownership. Journal of Vision 10(7), 1176-1176 (2010)

22. Froyen, V., Kogo, N., Feldman, J., Singh, M., Wagemans, J.: Integration of contour and skeleton based cues in the reconstruction of surface structure. Perception 40(Supplement), 175a(2011)

23. Gao, R.,Wu, T., Zhu, S., Sang, N.: Bayesian inference for layer representation with mixed markov random field. In:Energy Minimization Methods in Computer Vision and Pattern Recognition, pp. 213-224. Springer (2007)

24. Gibson, J.: The ecological approach to visual perception. Lawrence Erlbaum (1986)

25. Goldstein, E.B.: Sensation and perception, 6 edn. Pacific Grove CA: Wadsworth (2002)

26. Gousseau, Y., Morel, J.: Are natural images of bounded variation? SIAM journal on mathematical analysis 33(3), 634-648 (2001)

27. Hoiem, D., Efros, A., Hebert, M.: Recovering occlusion boundaries from an image. International Journal of Computer Vision 91 (3), 328-346 (2011)

28. Howard, I.: Perceiving in Depth, Volume 3: Other Mechanisms of Depth Perception, vol. 29. Oxford Univ Pr (2012)

29. Kanizsa, G.: Grammatica del vedere: saggi su percezione e gestalt, ii mulino (1980)

30. Kim, S., Feldman, J.: Globally inconsistent figure/ground relations induced by a negative part. Journal of Vision 9(10) (2009)

31. Kogo, N., Froyen, V., Feldman, J., Singh, M.,Wagemans, J.: Integration of local and global cues to reconstruct surface structure. Journal of Vision 11(11), article 1100 (2011)

32. Kogo, N., Galli, A., Wagemans, J.: Switching dynamics of border ownership: A stochastic model for bi-stable perception. Vision Research 51, 2085-2098 (2011)

33. Kogo, N., Strecha, C., Van Gool, L., Wagemans, J.: Surface construction by a 2-d differentiation-integration process: A neurocomputational model for perceived border ownership, depth, and lightness in kanizsa figures. Psychological review 117(2), 406 (2010)

34. Kopf, J., Cohen, M., Lischinski, D., Uyttendaele, M.: Joint bilateral upsampling. ACM Transactions on Graphics 26(3), 96 (2007)

35. Lee, S., Sharma, S.: Real-time disparity estimation algorithm for stereo camera systems. Consumer Electronics, IEEE Transactions on 57(3), 1018{1026 (2011)

36. Leichter, I., Lindenbaum, M.: Boundary ownership by lifting to 2.1 d. In: Computer Vision, 2009 IEEE 12th International Conference on, pp. 9-16. IEEE (2009)

37. Lindeberg, T.: Scale-space theory in computer vision. Springer (1994)

38. Liu, B., Gould, S., Koller, D.: Single image depth estimation from predicted semantic labels. In: Computer Vision and Pattern Recognition (CVPR), 2010 IEEE Conference on, pp. 1253-1260. IEEE (2010)

39. Maire, M.: Simultaneous segmentation and figure/ground organization using angular embedding. Computer Vision{ECCV 2010 pp. 450-464 (2010)

40. Marr, D.: Vision: A computational approach (1982)

41. Metzger, W.: Gesetze des sehens (die lehre vom sehen der formen und dinge des raumes und der bewegung). Frankfurt/M.: Kramer (1975)

42. Namboodiri, V., Chaudhuri, S.: Recovery of relative depth from a single observation using an uncalibrated (real-aperture) camera. In: Computer Vision and Pattern Recognition, 2008. CVPR 2008. IEEE Conference on, pp. 1-6. IEEE (2008)

43. Nitzberg, M., Mumford, D.: The 2.1-d sketch. In: Computer Vision, 1990. Proceedings, Third International Conference on, pp. 138-144. IEEE (1990)

44. Nitzberg, M., Mumford, D., Shiota, T.: Filtering, segmentation, and depth, vol. 662. Springer (1993)

45. Palou, G., Salembier, P.: Occlusion-based depth ordering on monocular images with binary partition tree. In: Acoustics, Speech and Signal Processing (ICASSP), 2011 IEEE International Conference on, pp. 1093-1096. IEEE (2011)

46. Parida, L., Geiger, D., Hummel, R.: Junctions: Detection, classification, and reconstruction. Pattern Analysis and Machine Intelligence, IEEE Transactions on 20(7), 687-698 (1998)

47. Paris, S., Durand, F.: A fast approximation of the bilateral filter using a signal processing approach. International journal of computer vision 81(1), 24-52 (2009)

48. Peterson, M., Skow, E.: Inhibitory competition between shape properties in figure-ground perception. Journal of Experimental Psychology: Human Perception and Performance 34(2), 251 (2008)

49. Petschnigg, G., Szeliski, R., Agrawala, M., Cohen, M., Hoppe, H., Toyama, K.: Digital photography with flash and no-flash image pairs. In: ACM Transactions on Graphics (TOG), vol. 23, pp. 664{672. ACM (2004)

50. Pham, T., Van Vliet, L.: Separable bilateral filtering for fast video preprocessing. In: Multimedia and Expo, 2005. ICME 2005. IEEE International Conference on, 4 pages. IEEE (2005)

51. Rensink, R., Enns, J.: Early completion of occluded objects. Vision research 38(15-16), 2489-2505 (1998)

52. Rubin, N.: Figure and ground in the brain. Nature Neuroscience 4, 857-858 (2001)

53. Saxena, A., Chung, S., Ng, A.: 3-d depth reconstruction from a single still image. International Journal of Computer Vision 76(1), 53-69 (2008)

54. Serra, J.: Image analysis and mathematical morphology, vol. 1. Academic Press (London and New York) (1982)

55. Soille, P.: Morphological image analysis: principles and applications. Springer-Verlag New York, Inc. (2003)

56. Tomasi, C., Manduchi, R.: Bilateral filtering for gray and color images. In: Computer Vision, 1998. Sixth International Conference on, pp. 839-846. IEEE (1998)

57. Torralba, A., Oliva, A.: Depth estimation from image structure. Pattern Analysis and Machine Intelligence, IEEE Transactions on 24(9), 1226-1238 (2002)

58. Vincent, L., Soille, P.: Watersheds in digital spaces: an efficient algorithm based on immersion simulations. IEEE transactions on pattern analysis and machine intelligence 13(6), 583-598 (1991)

59. Von Gioi, R., Jakubowicz, J., Morel, J., Randall, G.: Lsd: A fast line segment detector with a false detection control. Pattern Analysis and Machine Intelligence, IEEE Transactions on 32(4), 722-732 (2010)

60. Wang, J., Adelson, E.: Representing moving images with layers. Image Processing, IEEE Transactions on 3(5), 625-638 (1994)

61. Williams, L., Jacobs, D.: Stochastic completion fields: A neural model of illusory contour shape and salience. Neural Computation; Neural Computation (1997)

62. Yang, Q., Yang, R., Davis, J., Nistér, D.: Spatial-depth super resolution for range images. In: Computer Vision and Pattern Recognition, 2007. CVPR'07. IEEE Conference on, pp. 1-8. IEEE (2007)

63. Yaroslavsky, L.: Digital picture processing. An introduction., vol. 1. Springer-Verlag (1985)

64. Yu, S.: Angular embedding: from jarring intensity differences to perceived luminance. In: Computer Vision and Pattern Recognition, 2009. CVPR 2009. IEEE Conference on, pp. 2302-2309. IEEE (2009)

65. Zhou, H., Friedman, H., Von Der Heydt, R.: Coding of border ownership in monkey visual cortex. The Journal of Neuroscience 20(17), 6594-6611 (2000)

## _DESCRIPTION OF THE INVENTION_

The present invention focuses on recovering, in a single image_or a sequence of still images, relative depth from low-level features without explicit T-junction detection and interpretation. It is based on the extraction of low-level depth features to estimate a relative depth order for images (or parts of the images) that are focused, that is, where the imaged objects are inside the depth of field of the camera. It is assumed that only a single observation from an uncalibrated camera (unknown parameters) is available.

The approach in the present invention is more close to the Gestalt-based family, in the sense that a computational model for monocular depth perception is proposed. However, this model is not explicitly based on mid-level features that require detection stages or hard decisions, such as T-junctions, edges, or partition regions, and does not rely on the quality of the image segmentation or the edge detection. Instead, the proposed model is based on local low-level features that are directly computed on the image. Image segmentation or edge detection is not required, since it directly considers the connected components of the image as its fundamental units. This low-level model correctly interprets mid-level depth cues such as T-junctions and convexity, agreeing with human perception, as it will be later shown. This is the main difference of the current approach with respect to all Gestalt-based approaches and the main contribution of the present invention.

Although depth features are used at a different level, a similar methodology to that presented in [13] for the creation of the final depth map is followed. First, relative depth cues are locally extracted and, then, a global integration process is performed to provide a consistent depth order. In both cases, local information is propagated by non-linear diffusion, guided by color or gray level information. In [13] the diffusion process is implemented by a modified (maximum) Yaroslavsky neighborhood filter and, in the present invention, by a modified bilateral filter.

This methodology is not exclusive of the computer vision field. It has also been proposed in the context of psychology and perception. For instance, a similar approach is presented in [33], based on the global integration of local features. The local features are based on a hard interpretation of the T-junction candidates considered after an edge detection process. Global interaction is done through numerical integration of features along different paths on the image. The proposed model is applied to the completion of illusory contours and results are only provided for test images reflecting classical illusions. In [32], the model is modified, introducing some randomness in the low-level features to explain the foreground/background visual illusions. Other examples based on local propagation of depth cues are proposed in the context of relative depth ordering and border-ownership [20-22, 31]. The motivation for this strategy is based on psychophysical findings [30] suggesting that border ownership can vary locally along a boundary, even leading to a globally inconsistent figure/ground assignment. Moreover, this is also consistent with electrophysiological evidence showing local coding for border ownership in area V2 of the cortex as early as 68 msec after image onset [65]. This reinforces the low-level and local nature of monocular depth features and suggests a spatially distributed and potentially competitive process of figural assignment (global integration) [48], in which adjacent image areas compete to own their common boundary, with figural status propagating across the image as this competition proceeds.

The present low-level model has some connections with the perception model proposed in [61]. The present invention addresses the contour continuation problem, proposing a model based on the probability that a random walk from a termination reaches a certain pixel. Under this formulation, the maximum probability corresponds to the curve of least energy, in this case the elastica, that is, the curve that minimizes a linear combination of the length and the total (squared) curvature of the curve ($E = \alpha \int ds + \beta \int \kappa^2 ds, \alpha, \beta > 0$). The present model is based on the probability that a pixel belongs to a certain set (a connected component of a level set of the image), which in turn depends on the distance and the curvature of the distance function from the pixel to the set ($E = \alpha \cdot d - \beta \cdot |\kappa|^\gamma, \alpha, \beta, \gamma > 0$). The curvature term in [61] refers to the total (squared) curvature of the path and, hence, a path of minimum angular variation is more likely. In the present invention, the curvature term measures the difficulty of moving away from the set. In areas of null curvature (the boundary of the set is a straight segment), the cost of reaching a point outside the set is large since, to reach it, a large angular deviation is required. On the contrary, on areas of high curvature, the required change in direction is smaller. Thus, the curvature term in the present invention is related to the angular deviation needed to reach the point. Hence, a pixel is more likely to belong to a certain connected component if the local continuation from the boundary of the set to the pixel has a low energy. The energy depends on the length (in the present invention modeled by the distance from the set to the pixel) and the cumulated curvature of the path (reflected in the present model by the fact that at areas of high curvature a lower angular deviation of the path is required).

Under the above premises, the present invention defines local low-level features that encode perceptual monocular depth cues such as convexity/concavity, inclusion, and T-junctions in a quantitative manner. For that purpose, a novel computational model that agrees with the expected local responses of human perception is herewith presented. This model is not derived from edge or junction interpretation but from the properties of fundamental units such as the connected components of the level sets of the image intensity. Following a set occlusion model, connected components of level sets are considered as the visible parts of larger objects that have been occluded during the image formation process. The proposed low-level features are based on a measure of how likely is a pixel to belong simultaneously to several objects. Since a pixel is only visible in one of the sets, and occluded in the rest, the more sets the pixel is likely to belong, the higher the probability that there is an occlusion around it. This information provides a valuable hint on the local depth order of the pixel.

For each pixel, a membership measure proportional to the probability of belonging to a certain set is computed. This measure consists of two terms. The first term depends on the distance from the set to the pixel (the closer the pixel to the set, the more likely it belongs to it). The second term is based on a curvature measure of the boundary of the set, in practice the curvature of the level line of the distance function to the set at that pixel. Intuitively, assuming that the boundary of the original (non-occluded) sets is relatively smooth, points of high curvature are likely to be a consequence of an occlusion process. This measure also reflects the perceptual observation that convex objects are interpreted as being in the foreground. Summarizing, the pixels that are close to high curvature boundary points of a set are more likely to belong to the set than the rest. It will be shown that this basic model leads to accurate local depth cues that agree with the main principles of human perception.

Besides, there may be a relative weight between the two terms. These parameters can be chosen taking into account the local response of a T-junction (the size and decay of the response with respect to its center). As a consequence, a multi-scale low-level feature is defined, where the dependency of the model from the size of the response is removed, leaving the decay as the only parameter.

Once the local features have been determined, an integration mechanism is preferably used in order to assure global coherence and regularity of the relative depth map. This integration is performed by a non-linear diffusion process applied to the local depth information, guided by the color or gray level image data. More precisely, this diffusion process is preferably implemented by a modified bilateral filter [56], that averages the local depth data in the neighborhood of a pixel, weighted by the color or gray level similarity of the pixels [34, 62].

The present invention provides a computational model that encodes the principles of depth perception for objects in the depth of field, providing a (local) relative low-level depth feature that can be directly and efficiently extracted from images. The definition of multi-scale low-level features makes the relative depth analysis independent of the scale and decreasing the number of parameters of the model. This way, a measure of how likely is a pixel to belong simultaneously to different objects (interpreted as connected components of level sets) and, hence, to be occluded in some of them, is obtained, providing a hint on the local depth order relationships. The measures are directly computed on the discrete image data in an efficient manner, without requiring the detection and interpretation of edges or junctions. A mechanism to globally integrate the local low-level features is also employed in order to create a coherent and consistent depth order of the scene. The recovery of the relative depth order on the image is achieved by global integration of these local features applying a non-linear diffusion filtering of bilateral type. The validity of the proposed features and the integration approach is demonstrated by experiments on real images and comparison with state-of-the-art monocular depth estimation techniques.

The method for recovering a relative depth map from a single image or a sequence of still images comprises:

- decomposing the image into a plurality of sets $U_\lambda$, each one formed by the set of pixels whose intensity value is in a predefined intensity interval;

- decomposing each of the sets $U_\lambda$ into connected components $C_i^\lambda$, a connected component being defined as a subset of adjacent pixels according to a predefined pixel connectivity rule;

- for each connected component $C_i^\lambda$ of each set $U_\lambda$, computing membership measures indicative of the probability of each pixel x ∉ $x \notin C_i^\lambda$ to belong to said connected component $C_i^\lambda$; each membership measure being a function of:

  • the distance $d(x, C_i^\lambda)$ from the pixel x to said connected component $C_i^\lambda$;

  • the curvature $\kappa(x, C_i^\lambda)$ of the level line of the distance function $d(x, C_i^\lambda)$ from the pixel x to said connected

component $C_i^\lambda$ ;

- for each pixel $x$ of the image, using the previous membership measures to compute a local depth feature $Z(x)$ indicative of the probability of the pixel $x$ belonging simultaneously to different connected components and which provides a relative depth order estimation at pixel $x$.

In a preferred embodiment the method further comprises generating, for each pixel $x$ of the image, a multi-scale local depth feature $Z^{MS}(x)$ by combining weighted averages of the local depth features $Z(x)$ at different scales ($s_0$, $s_1$,..., $s_N$) to make the relative depth order measures independent of the scale. It may further comprise applying a global integration process to the multi-scale local depth features $Z^{MS}(x)$ to obtain a consistent relative depth map. The global integration process preferably comprises a non-linear diffusion guided by color or gray level information of the pixels, implemented by a modified bilateral filter that averages the multi-scale local depth features $Z^{MS}(x)$ in the neighborhood of a pixel $x$, weighted by the color or gray level similarity of the pixels.

For the generation of multi-scale local depth features $Z^{MS}(x)$ the weights $\omega(s_i)$ depending on the scale $s_i$ are preferably selected according to the size distribution of connected components $C_i^\lambda$ , giving more relevance to scales $s_i$ related to the size of the connected components with higher frequency of appearance on the image. The method may comprise computing the distribution of sizes of connected components $C_i^\lambda$ of the image to derive the weights $\omega(s_i)$. The weights $\omega(s_i)$ preferably have a decreasing behavior with increasing scales $s_i$ and are truncated or limited for lower scales $s_i$.

In a preferred embodiment, for the generation of multi-scale local depth features $Z^{MS}(x)$ the curvature $\kappa(x, C_i^\lambda)$ is computed after smoothing the distance function $d(x,\ C_i^\lambda\ )$ of the corresponding connected component $C_i^\lambda$ by a Gaussian kernel of standard deviation equal to the scale $s_i$ of analysis.

In another preferred embodiment, for the computation of a membership measure the method comprises computing a density of membership function $D(x, C_i^\lambda)$ indicative of the probability of the pixel x belonging to a certain connected component $C_i^\lambda$ , such that said function $D(x, C_i^\lambda)$ is increasing with the curvature $\kappa(x, C_i^\lambda)$ and decreasing with the distance $d(x, C_i^\lambda)$ from the pixel x to the connected component $C_i^\lambda$ . The density of membership function $D(x, C_i^\lambda)$ may be such that $D(x, C_i^\lambda) \propto e^{-E(x, C_i^\lambda)}$ , where $E(x, C_i^\lambda) = E_0(d(x, C_i^\lambda)) + E_1(\kappa(x, C_i^\lambda))$ is the energy of the pixel with respect to the connected component $C_i^\lambda$ , such that $E_0(d(x, C_i^\lambda))$ is increasing in $d(x, C_i^\lambda)$ and $E_1(\kappa(x, C_i^\lambda))$ is decreasing in $\kappa(x, C_i^\lambda)$.

In a preferred embodiment the local depth feature $Z(x)$ at a pixel x is obtained as the sum of the membership measures computed for said pixel x.

The intensity intervals may be defined as $[\lambda-\Delta/2, \lambda+\Delta/2)$, where $\Delta$ is a quantization step, and $\lambda \in \Delta Z$.

The pixel connectivity rule may be defined according to different rules, such as any of the following:

- in still images the adjacent pixels are the top, bottom, left, and right pixel neighbors;
- in still images the adjacent pixels are the top, bottom, left, right, top-left, top-right, bottom-left, and bottom-right pixel neighbors;
- in a sequence of still images, adjacent pixels are defined similarly as for still images but taking into account the time dimension;
- in a sequence of still images, the spatial connectivity in the time dimension is defined in terms of the optical flow, defining as adjacent in time the closest pixel indicated by the direction of the motion vector.

To summarize, the present invention proposes a new approach to extract low-level monocular depth information for depth-of-field objects of an image, in the form of a relative depth map. It is based on two stages:

- The computation of a set of low-level local features at multiple scales. The feature at each scale is based on computing a membership function for each pixel that is proportional to the probability of the pixel to belong to different connected components of the image. Hence, it encodes the likelihood that an occlusion process is taking place at that pixel.

In turn, the probability of a pixel to belong to a certain connected component depends on both: its distance to the set, and the curvature of the distance function at its location. In addition, the model parameters can be rewritten in terms of perceptual parameters such as the size of the response to a certain visual configuration. This provides further insight into the model and allows introducing the concept of scale in its computation.

-   An integration mechanism that ensures global consistency among the set of local low-level features. This mechanism is based on a non-linear diffusion process implemented by a neighborhood filter that propagates relative depth information, guided by the color or gray level information of the original image. It is simple and efficient, and it does not require any knowledge on the image content or any high level reasoning. Consequently, the overall approach can be considered as a low-level tool for monocular depth estimation.

The present invention has been evaluated in a large set of test and real images, demonstrating that it succeeds in most cases to estimate the relative depth maps for both types of examples. As it will been shown, the present approach outperforms state-of-the-art techniques, providing a finer and more accurate depth without requiring any mid-level analysis, such as segmentation, edge detection, or T-junction extraction.

The method proposed by the present invention supposes a contribution to the practical solution of the monocular depth estimation problem using low-level features. Although many relevant contributions have been made to this field since the pioneering work of Nitzberg and Mumford [43] in the 90's, they have not been able to encode in a simple and efficient manner the low-level process of monocular depth interpretation that takes place at early stages of the human visual system.

In fact, this may be the main reason why monocular depth cues, an important set of cues in human perception, have been mainly ignored in computer vision and image processing. Intermediate tasks, such as image segmentation or motion estimation, and high-level tasks, such as object detection or scene interpretation, can significantly benefit from this type of low-level information, increasing both their robustness and accuracy. The present method can be used to incorporate monocular depth features at the beginning of the image processing chain.

In that sense, the present invention aims at providing a fast implementation of the proposed model that can be computed in an efficient manner. This way, relative depth features could be extracted at the beginning of the image processing chain and exploited by higher level applications, without compromising its performance in terms of time and calculations.

The proposed method to estimate relative depth order in still images can be easily extended to video sequences, considering the video as a three-dimensional array data. In that case, the bi-levels and connected components are defined in three-dimensions using three-dimensional spatial connectivity. Optionally, the spatial connectivity in the time dimension can be defined in terms of the optical flow of the video sequence, computed by any of the state-of-the-art optical flow algorithms.

## BRIEF DESCRIPTION OF THE DRAWINGS

A series of drawings which aid in better understanding the invention and which are expressly related with an embodiment of said invention, presented as a non-limiting example thereof, are very briefly described below.

Figure 1 represents a case of study for a convex/concave boundary.
Figure 2 represents a case of study for a corner point.
Figure 3 represents a case of study for a T-junction configuration between three sets.
Figure 4 shows the results for a convex/concave set and its degenerate case, the corner.
Figure 5 shows the results for a T-junction.
Figure 6 shows the results for a set of aligned corners forming a cross.
Figure 7 shows the results for other test images with simple configurations.
Figures 8 to 11 shows the results for real images, and a comparison with the results obtained by four state-of-the-art monocular depth estimation techniques.
Figure 12 shows the results for real images.
Figure 13 compares the results of the present method with a particular relevant prior art technique.

## DESCRIPTION OF A PREFERRED EMBODIMENT OF THE INVENTION

The present invention proposes a new low-level computational model for monocular depth perception. Assuming that the image has been generated by a set occlusion model, it is considered the closed sets $X_1,...,X_n$ in $\Re^2$ and the partition of the plane given by

$$A_i := X_i \setminus \bigcup_{1 \le j < i} \text{int}(X_j) \tag{1}$$

where int($X$) denotes the interior of $X$. It is assumed that the sets $A_i$ have been thrown on the plane in the order $X_n,...,X_1$ and the set $A_i$ is the visible part of the set $X_i$. It may be called the occlusion set model. It can be considered as a realization of what is known in the literature as the dead leaves model [5, 54].

Further regularity on the sets $A_i$ shall be specified. For simplicity, it may be assumed that they have a piecewise smooth boundary.

For each $x \in \Re^2$, , $n(x, r)$ refer to the number of sets $A_i$, $i \in \{1,...,n\}$ such that $B(x, r) \cap A_i \ne 0$, and it may be called the multiplicity of x at the scale $r$, where $B(x,r)$ is a ball of center x and radius $r$, that is, the set B(x,r)={y, ||x-y||<r}, where $||\cdot||$ is the Euclidean norm. Notice that $n(x, r)$ decreases as $r \to 0 +$, so that x has multiplicity $n(x)$ if $n(x) = \lim_{r \to 0+} n(x, r)$. Clearly, if $n(x) = k$ then for some $R > 0$ we have $n(x, r) = k$ for all $0 < r < R$. Typically the multiplicity is 1, 2, 3. Generically, by slightly moving the sets we destroy points of multiplicity $\ge 4$ (sets are considered opaque). Thus, it may be assumed that $n(x) \in \{1,2,3\}$ for all $x$.

Clearly, points $x$ with $n(x) = 1$ are in the interior of the visible part of a set. Points $x$ with $n(x) = 2$ are on the common boundary of two sets. Points x with $n(x) = 3$ are a model for triple junctions and represent a strong index for occlusions. The problem may be stated as: "what is the order of the sets around $x$?". By answering this question, the sets may be locally ordered in depth.

Multiplicity and curvature are two key indexes that have been used for detecting triple junctions and giving a local depth order. This is essentially the program followed in [9, 10] where junctions were defined as points $x$ of multiplicity 3 and a measure of the curvature was given in terms of the angular aperture of each set $A$ at $x$ (measured as the area of $B(x,r) \cap A$). The occluding set was determined by the set with the largest angular aperture.

As in [32, 33], the principle of the present model is to detect depth cues, to compute and assign border ownership measures locally, and then integrate them in order to construct a depth ordering map (which of the sets is closer to the viewer). Knowing the object boundaries, the border ownership measure can be seen as a local concavity/convexity detector according to the perceptual observation that convex parts correspond to the owner side of the boundary. These local measures are integrated using a joint bilateral filter in order to compute a global depth ordering configuration [13,33]. This type of approach is followed in [32, 33] in order to reproduce the illusory perception of Kanizsa figures. The present invention shares this general outline, but has a different algorithmic approach in the details which enables to handle the case of real images. The methodology in the case of the simplified occlusion set model will be first explained, and then the case of real images will be discussed.

**Simplified occlusion set model: a border ownership model**

For each set $A \subset \Re^2$, $d(x, A)$ is defined as the distance from $x$ to the set $A$. Notice that $d(x, A) = 0$ if $x$ is in the closure of $A$. We denote by $\kappa(x,A)$ the curvature of the level line of the distance function $\{d(\cdot, A) = d(x,A)\}$, that is

$$\kappa(x, A) = div\left(\frac{\nabla d(x, A)}{|\nabla d(x, A)|}\right).$$ It may be implicitly understood that $\kappa(x, A) = 0$ if $x$ is in the closure of $A$. Finally, $A^c$ denotes the complement of $A$ and $\partial A$ denotes the boundary of A.

Function $D(x, A)$ is defined as a density of membership of the point x to the set $A$. In the context of the set occlusion model, the set $A$ is identified with the visible part of the object and we assume that $D(x, A) = 1$ if $x \in A$. Moreover, it is assumed that the sets have a piecewise smooth boundary.

A border ownership model based on a local convexity/concavity detector is used. If $A \subset \Re^2$ is locally convex around a point $x \in \partial A$, that is, if $A \cap B(x, r)$ is a convex set for some $r > 0$, then we assign the boundary $\partial A \cap B(x, r)$ to the set $A$. In case that $A^c \cap B(x, r)$ is a convex set, $\partial A \cap B(x, r)$ is assigned to $A^c$. This assignment is done through the border ownership density function $Z(x)$. In the case of a set A and its complement, we define

$$Z(x) = D(x, A) + D(x, A^c) \tag{2}$$

The measure $Z(x)$ depends on $A$, $A^c$ and represents a polarity model. It can be interpreted as an indication of a depth order between $A$ and $A^c$ around the point $x$. $Z$ must be such that if $x \in \partial A$ and $A \cap B(x, $ r$)$ is locally convex, then $Z$ is larger on $A \cap B(x, r)$ than on $A^c \cap B(x, r)$ (the $\partial A \cap B(x, r)$ belongs to the set $A$).

The density term $D(x, A)$ is defined phenomenologically based on two principles: (i) the lower the distance from a pixel $x$ to the set, the smaller the density of the configuration; and (ii) the larger its curvature $\kappa(x, A)$, the larger its density. Intuitively, the first principle states that the lower the distance of a pixel from the set, the more likely the pixel belongs to it. The second principle encodes the idea that the boundary of image objects holds some smoothness property, hence, points of large curvature along the boundary may be caused by occlusion. Consequently, pixels outside and close to them are more likely to have been occluded and its probability (or density measure) to be in the set increases. Since the curvature of the distance function $\kappa(x, A)$ depends on the curvature of the boundary of $A$, the density is larger for pixels near boundary points with large curvature values.

Thus, for $x \notin A$, a measure proportional to the probability of membership may be defined as:

$$D(x, A) \propto e^{-E(d, \kappa; A)} \,,\tag{3}$$

where $E(d, \kappa; A) = E_0(d(x, A)) + E_1(\kappa(x, A))$. Written in this way, the lower the energy of the configuration of a pixel with respect to a set, the larger its probability to belong to the set. By the properties required to $D(x, A)$, we assume that $E_0(d(x,A))$ is increasing in $d(x, A)$ and $E_1(\kappa(x, A))$ is decreasing in $\kappa(x, A)$.

As an example, it may be considered the following energy functions:

$$E_0(d) = \alpha d \,, \quad E_1(\kappa) = -\beta |\kappa|^{\gamma} \,,\tag{4}$$

where $\alpha, \beta, \gamma > 0$, with the abbreviation $d = d(x, A)$ and $\kappa = \kappa(x, A)$. Later it will be seen that is more convenient to replace $E_1(\kappa) = -\beta|\kappa|^{\gamma}$ by the measure

$$F(\kappa) = e^{-E_1(\kappa)} = e^{\beta |\kappa|^{\gamma}} - 1 \,.\tag{5}$$

In this case $F(\kappa) \sim |\kappa|^{\gamma}$ as $\kappa \to 0$ and the density is indifferent to straight line boundaries.

More generally, in the case of the set occlusion model the border ownership density function may be defined by

$$Z(x) = \sum_{i=1}^{n} D(x, A_i) \,.\tag{6}$$

When $n(x, r) = 2$, the behavior of $Z$ around $x$ assigns the boundary to the set which is locally convex in a neighborhood of $x$. Clearly when $n(x, r) = 3$, the behavior of $Z$ near $x$ is an index of possible occlusions and of the relative depth order of nearby sets. This behavior will be clarified by considering concrete examples. Before that, the definition of $Z(x)$ for real images will be considered.

**The case of real images**

Let $\Omega$ be the image domain and $u : \Omega \to \Re$ be the given image. To simplify the considerations, it may be assumed that $u$ is a continuous function. More generally, it may be assumed that $u$ is an upper semicontinuous function or a discrete image [11]. This permits to define the connected components of upper and lower level sets.

In the present case the following bi-level sets are defined:

$$U_{\lambda} = \left\{ x \in \Omega \mid u(x) \in \left[ \lambda - \frac{\Delta}{2}, \lambda + \frac{\Delta}{2} \right] \right\} \,,\tag{7}$$

where $\Delta$ is a quantization step, and $\lambda \in \Delta Z$ (the intervals may be also defined having some overlapping). When $\Delta = 1$, no quantization is applied, and the sets $U_{\lambda}$ correspond to the level lines of the discrete image, that is, the set of pixels with intensity value $\lambda$. The sets $U_{\lambda}$ may be directly the disjoint level sets (pixels of equal intensity) of the discrete image ($\lambda = \Delta = 1$); the disjoint level sets of a uniformly quantized version of the image ($\lambda = \Delta > 1$); or even non-disjoint overlapping sets ($\lambda < \Delta$). In the case of color images, instead of a scalar, $\lambda$ is a three-dimensional vector, $\lambda \in \Re^3$, for instance, in [R,G,B] coordinates.

The use of bi-level quantization with $\Delta > 1$ is aimed at reducing the computational time in real color images, since slight color variations due to blur and noise (even if it is of low-variance and even not noticeable) may generate a significant increase in the number of tiny connected components of the image that do not contain relevant depth information. The bi-level quantization is not intended as a naïve segmentation approach but as a simple denoising mechanism to reduce computational load of the algorithm. In that sense, if the quality of the image is high and it can be considered as free of noise and blur artifacts, bi-level quantization is not necessary at all.

Defining a set of overlapping bi-levels or using more sophisticated denoising techniques may be also applied for the same scope. Here, for the sake of simplicity, it is made clear that the performance of the method is quite insensitive to small noise perturbations or small quantization errors. By just applying such a naïve strategy and does not necessarily require more complex approaches.

We denote by $C_i^{\lambda}, i \in \{1, ..., N_C^{\lambda}\}$ the connected components of $U_{\lambda}$ ($N_C^{\lambda}$ is the number of connected components of $U_{\lambda}$). A connected component is a subset of adjacent pixels that belong to a given set. Two pixels are adjacent if they are neighbor pixels according to a predefined pixel connectivity. For instance, in still images (two-dimensional case) two types of connectivity are used to define the adjacent neighbors of a certain pixel: 4-connectivity (top, bottom, left, and right neighbors), and 8-connectivy (the previous four plus the neighbors in the diagonal directions: top-left, top-right, bottom-left, and bottom-right). In video, three-dimensional pixel connectivity is defined in a similar way, leading to 6-, 18- or 26-connectivity. Optionally, the spatial connectivity in the time dimension can be modified using a precomputed optical flow, defining as adjacent in time the closest pixel indicated by the direction of the motion vector. Connected components can be extracted by scanning the data and assigning or propagating labels according to the chosen connectivity. Without loss of generality and for the sake of simplicity, the two-dimensional case (still images) is considered in the mathematical developments presented here. Extension to higher dimensions is straightforward.

These connected components provide a decomposition of the image domain into disjoint sets:

$$\Omega = \bigcup_{\lambda} \left( \bigcup_{i=1}^{N_C^{\lambda}} C_i^{\lambda} \right) \tag{8}$$

Connected components of bi-level sets may be interpreted as visible pixels of a larger image unit or object. Due to the image formation process, the rest of the object has been occluded by other objects. $Z(x)$ may be defined as the sum of the membership measures corresponding to the partition of the image formed by all connected components of the bi-level sets $U_{\lambda}$. By analogy with statistical mechanics, we refer to $Z(x)$ as a partition function:

$$\hat{Z}(x) = \sum_{\lambda} \sum_{i=1}^{N_C^{\lambda}} e^{-E(x, C_i^{\lambda})} \ . \tag{9}$$

Since all pixels belong to the visible part of one of the connected components, that is, $x \in C_j^{\lambda}$ for some $j = j(x)$, and $e^{-E(x, C_j^{\lambda})}$ is a constant independent of x, we may redefine $Z(x)$ as

$$Z(x) = \sum_{\lambda} \sum_{\{i | x \notin C_i^{\lambda}\}} e^{-E(x, C_i^{\lambda})} \ . \tag{10}$$

The value of $Z(x)$ provides a quantitative measure of how likely is a pixel to belong simultaneously to different connected components. Or, in different words, it gives a hint on the local depth order relationship around the point x. For instance, if $Z(x) \approx 0$, the pixel x is likely to exclusively belong to its own connected component. That may be the situation of a pixel located in the inner part of a large connected component. On the other hand, $Z(x) > 0$ means that with certain probability the pixel may belong not only to its connected component but also to other image units.

The only reason for a pixel to belong to several connected components (discarding transparency effects) is occlusion: the pixel belongs simultaneously to its own visible connected component and, eventually, to other connected components that are not visible but occluded. Hence, the values of $Z$ around x provide a numerical indication of the possibility that an occlusion is occurring at x. In case of occlusion, the relative depth order means that the occluding object is closer to the observer than the occluded object. That means that large values of $Z(x)$ indicate that a pixel is closer to the observer than neighboring pixels with lower values of Z.

Summarizing, $Z(x)$ is a good low-level local feature for relative depth information. This behavior will be clarified by considering concrete examples where the behavior of $Z(x)$ is coherent with the perceptual response.

**Cases of study**

The behavior of $Z(x)$ for some generic cases, of interest in real images, is herewith analyzed. First, the behavior of $Z(x)$ around a point $x \in \partial A$, $A$ being locally convex/concave/flat around x. More generally, the case of an object that is contained into another. Then, the behavior of $Z(x)$ around corner points and T-junctions will be considered.

Convex/concave boundary between two sets

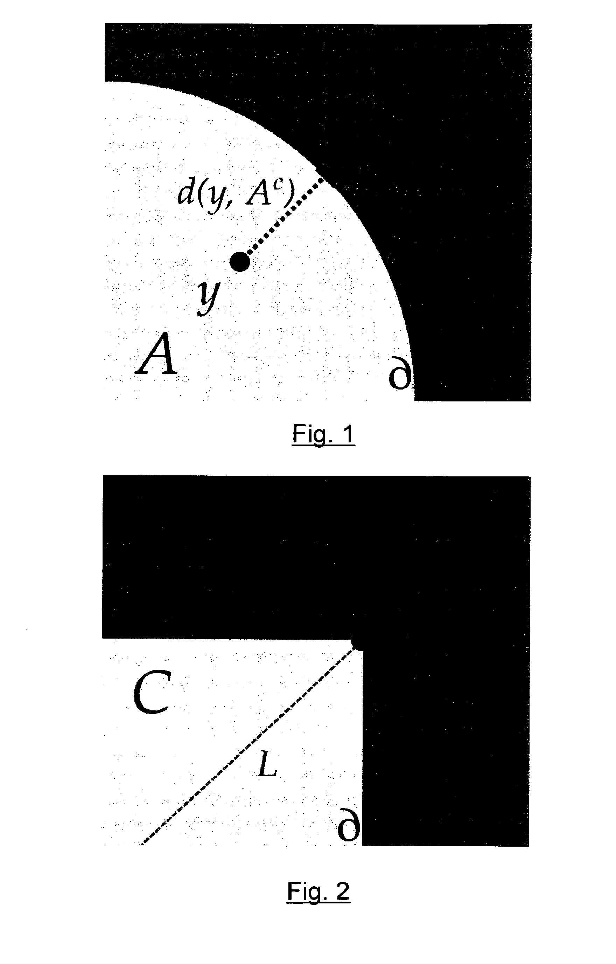

Considering a set $A \subset \Re^2$ such that $A \cap B(x, r)$ is convex and $\partial A \cap B(x, r)$ has bounded curvature (see **Figure 1**), by making r > 0 small enough it may assumed that $S_A(x) := \{y \notin \partial A : d(y, \partial A \cap B(x, r)) \leq r\}$ can be parameterized as $\pi_A(y) + tn_{\partial A}(\pi_A(y))$, where $\pi_A(y)$ is the unique point in $\partial A \cap B(x, r)$ such that $d(y, \partial A) = |y - \pi_A(y)|$, and $\mathbf{n}_{\partial A}(\pi A(y))$ is the inner unit normal to $\partial A$ at $\pi_A(y)$. Then

$$\widetilde{\kappa}(y, A) = \frac{\kappa_{\partial A}(\pi_A(y))}{1 + \varepsilon(y)\kappa_{\partial A}(\pi_A(y))d(y, \partial A)}; \qquad (11)$$

where $\widetilde{\kappa}(y, A)$ denotes the curvature of the curve $\{\hat{y} : d(\hat{y}, \partial A) = d(y, \partial A)\}$ at the point $y$, $\varepsilon(y) = 1$ if $y \in A^C$ $\varepsilon(y) = -1$ if $y \in A$.

Considering the energy $E_1(\kappa)$ given by equation (5), then for points $z \in A^c \cap B(x, r)$ we have

$$E(d(z, A), \kappa(z, A); A) = \alpha d(z, A) + E_1(\kappa(z, A)).$$

For *points* $y \in A \cap B(x, r)$ we have

$$E(d(y, A^C), \kappa(y, A^C); A^C) = \alpha d(y, A^C) + E_1(\kappa(y, A)).$$

Notice that if $d(z, A) = d(y, A^C)$ and $\pi_A(z) = \pi_A(y)$, then

$$E(d(y, A^C), \kappa(y, A^C); A^C) < E(d(z, A), \kappa(z, A); A),$$

since $E_1(\kappa)$ is a decreasing function of $\kappa$. Hence

$$Z(z) = D(z, A) + D(z, A^C) < D(y, A) + D(y, A^C) = Z(y).$$

Hence, for each point $y$ in the convex set $A \cap B(x, r)$, the equidistant point $z$ in $A^c \cap B(x,r)$ lying in the line perpendicular to the boundary and passing through $y$ has a lower value of $Z$ (see Figure 1).

As discussed before, if we consider the modified partition function as a relative depth feature (the larger the value the closer to the observer), and accumulate the values in both sides of the boundary of the convex set, using equation (11) the result is that

$$Z(A \cap S_A(x)) > Z(A^C \cap S_A(x)) \qquad (12)$$

and, hence, the convex set is interpreted as being in front of the concave set.

Equation (12) is justified since each $y \in A \cap S_A(x)$ can be written as $y = y(\theta) + tn\partial_A(y(\theta))$, where $y(\theta) = \pi_A(y) \in \partial A \cap B(x,r)$ is the point whose unit normal makes the angle $\theta \in [\alpha, \beta];]$ with $\mathbf{n}_{\partial A}(x)$, and $t = d(y, \partial A) \subset [0, r]$.

Let us denote by $\widetilde{\kappa}(\theta, t, X)$ the expression given in equation (11) when $y \in X$, where $X = A, A^C$. Let $F(\widetilde{\kappa}(\theta, t, A))$

be given by equation (5). Thus, in the continuous version, we may write

$$Z(A \cap S_A(x)) = \int_\alpha^\beta \int_0^r e^{-\alpha t} \frac{F(\widetilde{\kappa}(\theta, t, A))}{\widetilde{\kappa}(\theta, t, A)} dt d\theta \ .$$

Similarly, we have

$$Z(A^C \cap S_A(x)) = \int_\alpha^\beta \int_0^r e^{-\alpha t} \frac{F(\widetilde{\kappa}(\theta, t, A^C))}{\widetilde{\kappa}(\theta, t, A^C)} dt d\theta \ .$$

By the particular choice of $F(\kappa)$, the function $F(\kappa)\kappa^{-1}$ is an increasing function of $\kappa$. Since $\widetilde{\kappa}(\theta, t, A^c) < \widetilde{\kappa}(\theta, t, A)$, equation (12) can be deduced.

This local interpretation agrees with the perceptual principles of human vision [29], where convexity is an important local monocular depth feature to determine that an object occludes another one.

In the previous configuration, where we have considered that the locally convex set $A$, the curvature $\widetilde{\kappa}(y, A)$ explodes as $y \in A$ tends to the center of the osculating circle at $x = \pi_A(y)$ i.e. the point $x + \kappa_{\partial A}(x)\mathbf{n}_{\partial A}(x)$, where $\mathbf{n}_{\partial A}(x)$ denotes the inner unit normal to $\partial A$ at $x$. Those points belong to the morphological skeleton of $A$ and have energy $Z(x) \rightarrow +\infty$. That means that a pixel in the skeleton of a convex set has locally a null probability to belong to any other set. Hence, the skeleton in the inner part of a locally convex set reinforces the interpretation that the convex set is in front of the concave set. From a psycho-visual perspective, the skeleton can be related to convexity, inclusion, and closure perceptual depth cues. Notice that, in practice, curvature values computed over discrete images are bounded.

Straight boundary

Let us use again the energy density given by

$$D(x, A) = e^{-E_0(d(x,A))} F(\kappa(x, A)), \tag{13}$$

where $F(\kappa+)$ is given by equation (5). If $\partial A$ is locally a straight line, then the level lines of the distance function induced by any of the two sets $A, A^c$ are also locally straight lines and its curvature is null. Thus, for two points $a \in A$ and $b \in A^C$ lying on a perpendicular line to the boundary and being equidistant to it, we have $D(a, A^C) = D(b, A) = 0$, and so does the modified partition function $Z(a) = Z(b) = 0$.

In this situation, the proposed model does not favor any of the sets with respect to the other, which means that the sets are estimated to be at the same depth or, at least, that nothing can be said about its relative depth order using only local information. This behavior agrees with the human perception principles established in [29].

Corner points

For simplicity let us consider an infinite corner $C$ (see **Figure 2**). Let $x$ denote the corner point. As in equation (11), let $\widetilde{\kappa}(y, C)$ be the curvature of the level line $\{\hat{y} : d(\hat{y}, \partial C) = d(y, \partial C)\}$ at the point $y$. Let $L$ be the half-line contained in $C$ and bisecting the corner. If $y \in L$, then $\widetilde{\kappa}(y, C) = +\infty$, while $\widetilde{\kappa}(y, C) = 0$ if $y \in C \setminus L$. Let $N(x)$ be the outer normal cone at the point x. Clearly, $\widetilde{\kappa}(y, C) = 0$ if $y \in C^c \setminus N(x)$ and $\widetilde{\kappa}(y, C) = \frac{1}{|y - x|}$ if $y \in N(x)$. For simplicity we can consider a regularized version of C, obtaining

$$Z(C \cap S_C(x)) > Z(C^C \cap S_C(x)), \tag{14}$$

where $S_C(x) = \{y \notin \partial C : d(y, \partial C \cap B(x, r)) \le r\}$ for some $r > 0$. There is an accumulation of curvature in the skeleton of C (although the discretization of the curvature operator gives a curvature in the interval [-4; 4]). Corners favor the configuration where the convex set $C$ is in front of the concave set $C^c$.

The previous interpretation agrees with the psycho-visual role that corners play as convexity cues (in convex/concave configuration). In other situations they appear as forming part of T-junctions which are a powerful cue for occlusions. Later, we will examine the response of detector $Z(x)$ in that case.

<u>T-junctions</u>

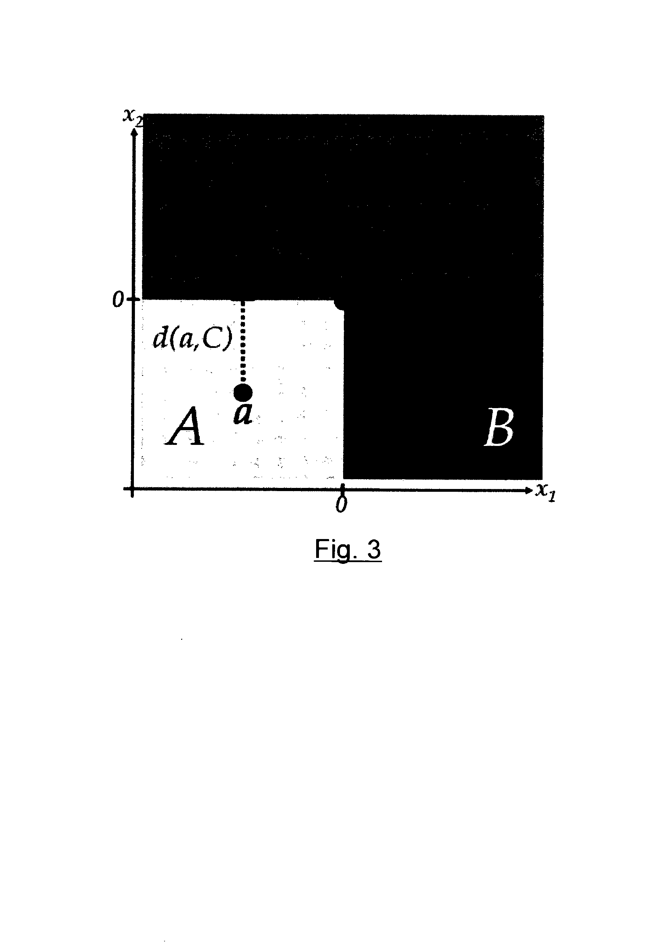

The configuration under study is the T-junction (see **Figure 3).** In most situations this configuration is formed when two sets are occluded by a third one, and that is why it is perceived as a strong cue for monocular depth. The perceptual interpretation at a local scale is that the set that owns the horizontal top segment of the "T" is in front of the two other sets forming the vertical segment of the "T". Considering the sets in Figure 3, the perceptual response triggered by the T-junction establishes that the set $C$ is occluding the sets $A$ and $B$, being relatively closer to the observer than the other two. The depth order of sets $A$ and $B$ cannot be established and it can be assumed that they are at the same depth.

Let us consider two points $a \in A$, $c \in C$, lying on a line perpendicular to the boundary of both sets and such that $d(a,C) = d(c, A)$, (we avoid points in the axis $x_1 = 0$). Let us give conditions on the parameters that imply that $Z(c) > Z(a)$. By symmetry, the same conditions apply to a similar configuration considering points in $B, C$.

Without loss of generality consider that the junction point is located at $x = 0$. Clearly, since $F(\kappa(y, C)) = 0$ for all $y \in C^c$, we have

$$D(y,C) = 0 \quad \forall y \in C^C.$$

If $y \in A^c$,

$$D(y,A) = \begin{cases} 0, & y \notin N_A(x) \\ e^{-\alpha|y|}(e^{\beta\frac{1}{|y|^\gamma}} - 1), & y \in N_A(x) \end{cases}$$

and if $y \in B^c$,

$$D(y,B) = \begin{cases} 0, & y \notin N_B(x) \\ e^{-\alpha|y|}(e^{\beta\frac{1}{|y|^\gamma}} - 1), & y \in N_B(x) \end{cases}$$

Where $N_A(x), N_B(x)$ denote the normal cones to the sets $A$, B at the point $x$, respectively. This implies that (notice that the density of belonging to the own set was discounted)

$$Z(a) = 0, \tag{15}$$

and

$$Z(c) = e^{-\alpha|c|}(e^{\beta\frac{1}{|c|^\gamma}} - 1), \tag{16}$$

Hence $Z(c) > Z(a)$. Taking local averages it is concluded that $Z(C \cap B(x, r)) > Z(A \cap B(x, r))$, $Z(B \cap B(x, r))$ and we deduce that $C$ is locally above $A$ and $B$ around the T-junction $x$.

<u>Model Parameters in terms of Perceptual Response to Basic Configurations</u>

As it has been shown, the relative depth feature provided agrees with the perceptual interpretation of the studied configurations. Notice that the proposed model has three parameters, $\alpha$, $\beta$ and $\gamma$, with the only condition of being positive. We can fix the model parameters in terms of size and decay of the perceptual response to a convex/concave or a T-junction configuration.

Let us start by introducing the decay condition on the T-junction response. This condition can be also imposed in the convex/concave case but for the sake of brevity we will focus on the T-junction configuration. For that, we consider the T-junction configuration displayed in Figure 3. Let us consider a point $y \in C$. Assume that $y \in N_A(x)$, if $y \in N_B(x)$, we proceed in a similar way. We measure the decay of $Z(y)$ with respect to the response $Z(y_0)$ where $y_0$ is near $x = 0$, the center of the T-junction. In practice, since we work at the discrete level, $y_0$ will be a pixel neighbor to $x$ (we take a nearest neighbor). We identify the scale s of the junction by the neighborhood $\{y : \frac{|y|}{|y_0|} \le s\}$. If we fix the scale of the junction, the response $Z(y)$ at $y$ such that $\frac{|y|}{|y_0|} = s$ decays an order of magnitude with respect to $Z(y_0)$, that is

$$Z(y_0) = kZ(y),\qquad(17)$$

where, in practice, $k = 10$. At each scale of analysis, we choose a set of parameter values for which this equation has a solution.

Notice that there are three parameters and equation (17) is a single constraint, thus this represents a surface in the space of parameters $\alpha$, $\beta$ and $\gamma$. For the moment, assume that $\gamma > 0$ is fixed. Then writing $a = \frac{1}{|y_0|^{\gamma}}$, $b = \frac{1}{|y|^{\gamma}}$, we can rewrite equation (17) as

$$ke^{\alpha(a^{-1/\gamma} - b^{-1/\gamma})} = \frac{e^{a\beta} - 1}{e^{b\beta} - 1}.\qquad(18)$$

Notice that, being $a > b$, $f(\alpha) := ke^{\alpha(a^{-1/\gamma} - b^{-1/\gamma})}$ is a decreasing function of $\alpha$. On the other hand, we have:

**Lemma 1**. Assume that $a > b$. The function $g(\beta) := \frac{e^{a\beta} - 1}{e^{b\beta} - 1}$ is an increasing function of $\beta$. Since $f(\alpha)$ is decreasing, $g(\beta)$ is increasing, and $f(0) = k, g(0) = \frac{a}{b} > 1$, it $\frac{a}{b} < k$, we have that for each $\alpha$ such that $\frac{a}{b} \le f(\alpha) \le k$, there is a value of $\beta$, and only one, such that b $f(\alpha) = g(\beta)$ The interval of $\alpha$ can be expressed as $[0, \alpha^*]$, where

$$\alpha^* = f^{-1}(g(0)) = \frac{\ln(a/(kb))}{a^{-1/\gamma} - b^{-1/\gamma}}.$$

In practice we take $|y_0| = 1$, thus $a = 1$, and $\frac{a}{b} < k$ can be written as

$$|y| \le k^{1/\gamma} = 10^{1/\gamma}.\qquad(19)$$

Having fixed the scale $s$, we should have

$$s \le 10^{1/\gamma}.\qquad(20)$$

This condition imposes a constraint in the value of $\gamma$. Since we are going to consider a range of scales between 1 and 40, we choose a value of $\gamma = 0.6$. Then equation (20) holds and we can find values of $\alpha$, $\beta$ for which equation (18) holds. These values can be numerically pre-computed and stored into a look-up-table. An example of look-up-table for some configurations is outlined in Table 1 below (example of look-up table of pre-computed model parameters $(\alpha, \beta, \gamma)$ at different scales $(s)$ for a response decay of $k = 10$ (and $|y_0| = 1$)). In this way, when the algorithm is executed, it can load the values of the model parameters without spending any extra time in its computation.

*Table 1*

| Scale ($s$) | $\alpha$ | $\beta$ | $\gamma$ |
|---|---|---|---|
| 2 | 1.4901 | 1.8677 | 0.6 |
| 3 | 0.6980 | 0.9192 | 0.6 |
| 4 | 0.3737 | 1.0964 | 0.6 |
| 5 | 0.2593 | 0.8796 | 0.6 |
| 6 | 0.1932 | 0.7341 | 0.6 |
| 7 | 0.1625 | 0.4432 | 0.6 |
| 10 | 0.0608 | 0.9123 | 0.6 |
| 15 | 0.0206 | 0.8910 | 0.6 |
| 20 | 0.0121 | 0.6223 | 0.6 |
| 25 | 0.0044 | 0.5884 | 0.6 |
| 30 | 0.0042 | 0.3127 | 0.6 |

Summarizing, thanks to equation (17), at each scale s we can find (and fix) values of the parameters $\alpha$, $\beta$, $\gamma$ for which the response function has a specified decay. In practice, the value of $k$ can be set to assure that the response is concentrated in the area around the studied configuration (similarly to the bandwidth of a filter).

For instance, a value of $k$ = 10 (decay of one order of magnitude) is considered in examples later shown. The scale parameter $s$ defines the size of the extracted local low-level feature. One option is to set this parameter to a proper value that captures most depth information present in the image. Another possibility is to consider not a single but a set of relevant scales and to define a multi-scale low-level feature, as it will be now explained.

**Multi-scale low-level local features**

The values of parameters $\alpha$, $\beta$ have been fixed depending on the scale $s$ and the size $k$ of the response of the feature $Z$ at a T-junction configuration. Nevertheless, instead of selecting a single value of $s$, it is here proposed to generate a multi-scale low-level local feature combining the local features $Z$ at different scales.

The benefit of this approach is twofold: first, gathering information from different scales provides a much richer low-level feature and increases its robustness against noise; and second, the dependency of the model on the scale of analysis is removed, being left with a single parameter (the value of the decay $k$).

Hence, a multi-scale low-level feature $Z^{MS}(x)$ is defined as a weighted average of the normalized features at different scales $\{s_0, s_1,..., s_N\}$:

$$Z^{MS}(x) = \frac{1}{\Omega} \sum_{i=1}^{N} \omega(s_i) \frac{Z_{s_i}(x)}{\max_y [Z_{s_i}(y)]},$$

(21)

where $\Omega = \sum_{i=1}^{N} \omega(s_i)$. The features at each scale are divided by its maximum value and, hence, the value of $Z^{MS}$ at any pixel belongs to the interval [0, 1].

The value of the feature at scale $s_i$ is given by equations (10), (13):

$$Z_{s_i}(x) = \sum_{\lambda} \sum_{\{i | x \notin C_j^\lambda\}} e^{-E_{0s_i}(x, C_j^\lambda)} \cdot F_{s_i}(x, C_j^\lambda),$$

(22)

with

$$E_{0s_i}(x, C_j^{\lambda}) = -\alpha_i \cdot d(x, C_j^{\lambda}),$$

$$F_{s_i}(x, C_j^{\lambda}) = e^{\beta_i \left| \kappa_{s_i}(x, C_j^{\lambda}) \right|^{\gamma}} - 1$$

where the model parameters $\alpha$, $\beta$, $\gamma$ may have a different value depending on the scale (as discussed before and shown in Table 1) and curvature term

$$\kappa_{s_i}(x, C_i^{\lambda}) = div\left( \frac{\nabla(G_{s_i} * d(x, C_j^{\lambda}))}{\left| \nabla(G_{s_i} * d(x, C_j^{\lambda})) \right|} \right) \tag{23}$$

is computed after smoothing the distance function of the corresponding connected component by a Gaussian kernel of standard deviation equal to the scale of analysis. Smoothing the distance function by a kernel with size comparable to the scale of analysis assures that noise and small structures that are not relevant at the current level of detail are essentially removed and do not contribute to the local feature. The consequence is that the larger the scale, the more robustness to noise but the less discrimination in terms of depth resolution.

Finally, it is important to discuss the choice of weights depending on the scale. The simplest choice would be to take uniform weights in a predefined discrete set of scales ($s \in \{2,3,...,20\}$) independent of the image. Although this works for simple test images, a better approach for real images is obtained if one takes into account the distribution of objects at each scale. In this way, more importance is given to scales that are related to the size of the connected components with higher frequency of appearance on the image.

The size distribution of connected components of image level sets has been the object of study in [2, 26]. In these papers, the authors found that the empirical distribution of areas of the connected components of bi-level sets has a density function

$$f_A(a) = \frac{C}{a^{\delta}},$$

where the area $a$ varies in a certain range [$a_{min}$, $a_{max}$] This law was found to be adapted for natural and synthetic images, and for different types of textured images. It has been observed that for natural images the typical exponent is around 2, this implying that natural images with infinite resolution cannot be modeled by functions of bounded variation [2, 26]. In the case of synthetic images generated by the superposition of simple shapes like disks, rectangles or polygons, the exponent is around 1. In particular, for disks, $\delta = 0.8$. This is the case of interest. Indeed, the feature $Z_s(x)$ (at the scale $s$) has only sense if the objects around $x$ have at least a scale of $s$. This would be connected to the statistics of sizes of objects (or connected components of bi-level sets) that are dilated by balls of radius $s$. For that reason, the exponent $\delta = 0.8$, corresponding to images generated by disks, is preferably used. According to [2], we can assume $C \approx 10$. According to this, the weights are given by

$$\omega(s_i) = \frac{C}{(\pi s_i^2)^{\delta}}$$

But, the lowest scales suffer more severely from noise since they gather information of small structures. Taking this into account, the importance of lower scales is decreased by truncating the weights

$$\omega(s_i) = \min\left( \frac{C}{(\pi s_i^2)^{\delta}}, \frac{C}{(\pi s_{min}^2)^{\delta}} \right), \tag{24}$$

where $s_{min} = 5$. In other words, it is presumed that the most relevant image structures lie at intermediate levels of detail, and, therefore, medium scales represent the more relevant contributions since they are more robust to noise (the features being computed after filtering with a Gaussian). The weight function has a decreasing behavior with the scale, leading to a negligible contribution for very large scales. This behavior agrees with the idea that, after a certain point, very large scales contain few information and too low depth resolution. In addition, the decay of the contribution bounds the number of scales that have to be computed to build the multi-scale low-level depth feature. In practice, only those scales whose contribution to the weighted is above a certain threshold are computed. In different experiments performed, only the scales holding $\omega(s_j) > 0.001$ were considered.

Notice that the multi-scale feature can be computed in an efficient manner. Taking into account that the order of the gradient operator and the convolution by the Gaussian kernel $G_s$ in equation (23) can be inverted, the distance function and its gradient are only computed once for each connected component. Then, thanks to the additivity property of the Gaussian scale-space [37], this convolution can be efficiently implemented by convolving iteratively with a smaller Gaussian kernel. The process can be further accelerated and the amount of memory reduced using a multi-resolution approach implemented by a Gaussian pyramid.

As an alternative, one could compute the distribution of sizes of connected components of level sets for each given image and derive an expression (image dependent) for the weights $\omega(s_j)$.

**Global Integration of Low-Level Relative Depth Local Features**

Due to the local nature of the low-level features presented, their response is concentrated in specific areas of the image with relevant relative depth order information. Hence, it is necessary to propagate this information inside its corresponding image region or object. On the other hand, the different values of the responses may require a homogenization, and even may contradict each other presenting inconsistencies in the depth order. Thus, a non-local integration mechanism is necessary to assure coherent depth information inside the object. For that reason, once the local low-level features have been extracted, an integration strategy that provides a globally consistent depth map is applied.

Ideally, we should integrate local depth information coming from the same object and propagate it inside. The main difficulty is how to determine which pixels belong to the same object. As in [1, 12, 14, 15, 23], one could apply an image segmentation technique and use the resulting regions as integration support. Nevertheless, image segmentation is a mid- or high-level process (depending on the features used) and a difficult task itself, that usually requires hard decisions to compute the image partition. It is not clear how over-segmentation and, especially, sub-segmentation errors would affect the relative depth map.

In order to avoid using image segmentation or the result of any other mid- or high-level task, the present invention proposes to use a neighborhood filter, similar to the integration approach proposed in [13]. Unlike [13], we apply a bilateral filter [56] to integrate the local depth features on its neighborhood, but using the original color or gray level image information to determine the adaptive neighborhood as in the joint bilateral filter [16, 49]. In other words, depth information is averaged on a pixel neighborhood using color-based weights. Thus, given the low-level depth features $Z^0(x) = Z^{MS}(x)$ and the original image $I(x)$, the integrated features $Z^1(x)$ are computed as:

$$Z^1(x) = \frac{1}{W(x)} \sum_{y \in N(x)} w(x,y) \cdot Z^0(y), \tag{25}$$

where

$$W(x) = \sum_{y \in N(x)} w(x,y), $$

and the weights $w(x, y)$ are given by

$$w(x,y) = e^{\frac{|x-y|^2}{\sigma_s^2}} \cdot e^{\frac{|I(x)-I(y)|^2}{\sigma_c^2}} \tag{26}$$

with $\sigma_s, \sigma_c > 0$. These two parameters control the size (in terms of spatial and color coordinates) of the neighborhood of x that has influence to determine the value of $Z^1(x)$. This modified version of the bilateral filter has been already applied in the context of depth enhancement and super-resolution [34, 62].

The bilateral filter specified by equation (25) can be iterated several times (or until convergence)

$$Z^{n+1}(x) = \frac{1}{W(x)} \sum_{y \in N(x)} w(x,y) \cdot Z^n(y) \qquad (27)$$

to obtain an estimate of relative depth order. We denote by $Z^d(x)$ the final estimated value, expressing the relative depth order.

Equation (27) is a weighted average iterative filter with fixed weights, since the value of $w(x; y)$ only depends on the original image, I(x), and does not change from iteration to iteration. This permits to improve the efficiency of the global integration at the cost of an increase of the memory of the system. Since the weights are not modified, they can be initially computed and stored for the next iterations. However, assuming that the size of the neighborhood around each pixel is $m$ x $n$, this requires an additional memory of $m \cdot n$ times the size of the image.

**Experimental Results**

Test images

A series of test figures are examined in order to provide more intuition and understanding of the proposed low-level depth features and their global integration mechanism. In all examples, multi-scale features described in Equation (21) have been computed on a predefined discrete set of scales ($s \in \{2,3,...,20\}$), using uniform weights. The uniform quantization step was set to $\Delta = 1$ for gray level values in the interval [0,255]. The parameters of the bilateral filter for the multi-scale feature integration are $\sigma_s = 5$ , $\sigma_c = 3/255$ , for test images with an approximate size of 200 x 200 pixels. All relative depth maps shown correspond to the result of 100 iterations of the bilateral filter, and are normalized to [0, 1] with the convention that the brighter the value of a pixel, the closer to the observer, and viceversa.

It is important to recall that the depth information obtained is relative, meaning that it has to be interpreted in terms of depth relations among neighboring areas of the image. For instance, the fact that two regions that are not adjacent have the same value of $Z^{MS}(x)$ does not imply that both regions are at the same absolute depth. In that sense, the monocular depth information provided has to be interpreted in the context of the image region or object and its surrounding neighbors.

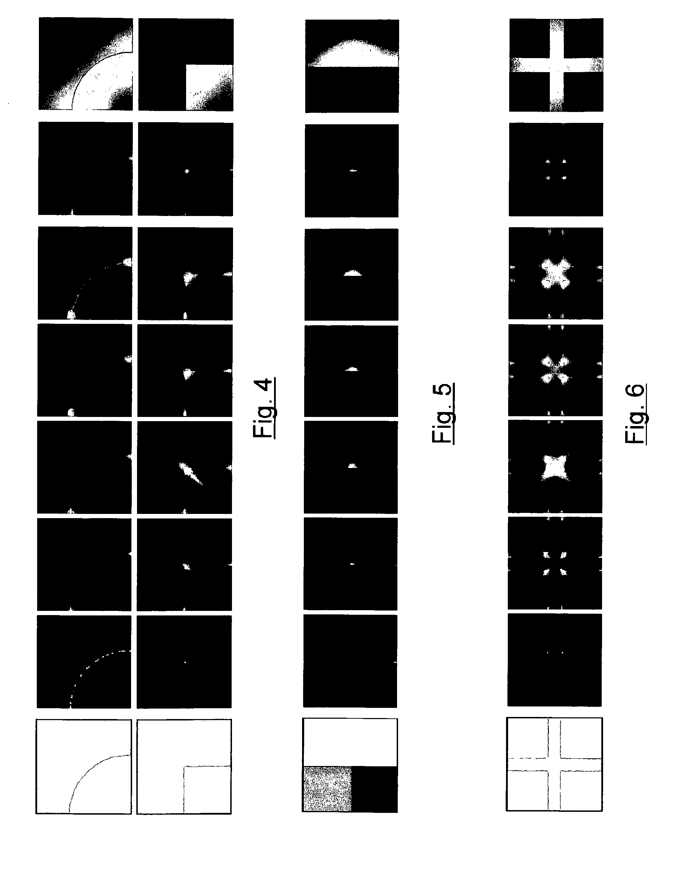

**Figures 4** and **5** present the results for the basic configurations previously studied. Figure 4 shows convex/concave sets and its degenerate case, the corner. For each row, columns from left to right:

- original image;
- features at scale $s = 4$;
- features at scale $s = 8$;
- features at scale $s = 12$;
- features at scale $s = 16$;
- features at scale $s = 20$;
- multi-scale features (equation (21), $s = 2, 3,..., 20$);
- global feature integration after 100 iterations (fine relative depth map).

The parameters for the bilateral filter: $\sigma_s = 5$, $\sigma_c = 3/255$.

Figure 5 shows an analysis of a T-junction. For each row, columns from left to right:

- original image;
- features at scale $s = 4$;
- features at scale $s = 8$;
- features at scale $s = 12$;
- features at scale $s = 16$;
- features at scale $s = 20$;
- multi-scale features (equation (21), $s = 2, 3, ..., 20$);
- global feature integration after 100 iterations (fine relative depth map).

Bilateral filter with $\sigma_s = 5$ , $\sigma_c = 3/255$.

For two sets sharing a convex/concave boundary, the features obtained at different scales and their combination agree with the convex set being closer to the observer than the concave set, as expected from the model.

For the corner, which is the degenerate case of the previous one, a similar behavior is observed. However, note that in this case the high curvature point (the corner itself) also induces some energy in the concave set. On one hand, it means that in the present model the corner is a weaker cue to establish the depth relationship between the two sets than a smoother convex/concave boundary, since the relative difference between both sets becomes smaller (although the convex set is still in front of the concave). On the other hand, the presence of relatively more energy in the concave set can be interpreted as a cue of occlusion in the form of a degenerate T-junction.

In other words, an isolated corner is interpreted similarly to a convex/concave boundary, but aligned with other corners it can be interpreted as part of an occlusion process. An example of this situation is shown in **Figure 6** with a set of aligned corners forming a cross. For each row, columns from left to right:

- original image;
- features at scale $s$ = 4;
- features at scale $s$ = 8;
- features at scale $s$ = 12;
- features at scale $s$ = 16;
- features at scale $s$ = 20;
- multi-scale features (equation (21), s = 2, 3, ..., 20);
- global feature integration after 100 iterations (fine relative depth map).

Bilateral filter with $\sigma_s$ = 5, $\sigma_c$ = 3/255.

Note that the cross is perceived as in front of the background formed by the set of corners. The previous reasoning is consistent with the perceptual theory presented in [29], where a set of corners can launch a modal or amodal completion mechanism that leads to the perception of illusory contours.

In Figure 5, results obtained for the T-junction configuration are shown. At lower scales, the information is very well localized (for instance, $s$ = 4) but the depth interpretation is more ambiguous. Higher scales have a lower resolution in terms localization (that is, detecting the exact point of the T-junction) but in contrast they provide a clear interpretation in terms of depth order. The weighted combination of the scales provides a multi-scale feature that combines both: a good localization and a reliable depth cue. After the integration process, the method provides a fine depth map with a correct depth order: the white region is in front of the gray and dark regions, while those two regions are set to the same relative depth.

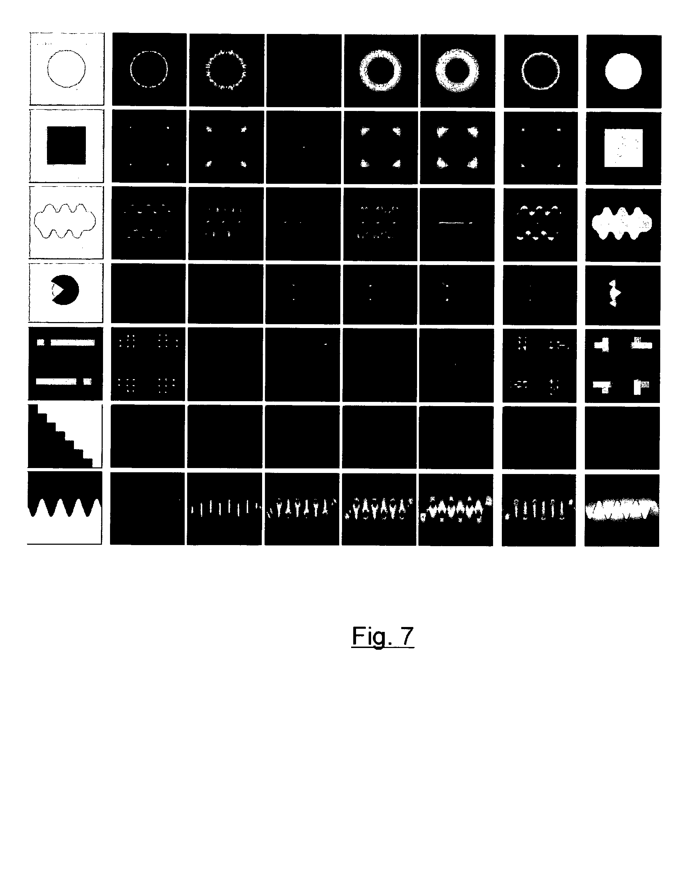

Other test images with simple configurations are presented in **Figure 7.** For each row, columns from left to right:

- original image;
- features at scale $s$ = 4;
- features at scale $s$ = 8;
- features at scale $s$ = 12;
- features at scale $s$ = 16;
- features at scale $s$ = 20;
- multi-scale features (equation (21), $s$ = 2, 3,..., 20);
- global feature integration after 100 iterations (fine relative depth map).

Bilateral filter with $\sigma_s$ = 5 , $\sigma_c$ = 3/255.

For the circle (first row) and the square (second row), it can be observed that the complementary information provided by each scale agrees with the interpretation of both figures being in front, and so does the multi-scale feature. Note that, in the case of the square, no depth information is provided in straight boundaries by the lower scales. In both examples, it is remarkable that the image features have energy not only on the boundary between the main set and the background, but also on the interior of the object. This inner energy corresponds to points of high curvature of the distance function (infinite in theory, although it is bounded in practice for discrete images) at the skeleton of the circle and the square. Notice also that this information appears as the more relevant at some specific scales. In particular, notice the high energy at the center at the scale $s$ = 12. In terms of depth cues, the information provided by the center is not related to convexity or occlusion, but to other cues such as inclusion or closure.

A similar conclusion can be extracted from the example shown in the third row of Figure 7, which corresponds to a close shape with a sinusoidal boundary. It can be seen that the points of high curvature of the distance function to the boundary correspond to the (external and internal) skeleton of the shape [55] and significantly contribute to the energy of the features at different scales (being especially relevant at some of them). As above, this contributes to introduce energy in the interior of the object, and also to speed-up the convergence of the integration algorithm to finally obtain a relative depth map with the object in front of the background.

The example in the fourth row of Figure 7 corresponds to a "pacman" configuration. It can be seen that in this situation some of the features are contradictory. For instance, at most of the scales and at their combination, the T-junction generated by the white and gray objects together with the straight boundary of the black object favors the interpretation that, locally, the black object is in front of the other two. Nevertheless, the sharp corner of the white object close to the center of the black object (the white object being here locally the convex set and the black object being the concave one) provides the opposite interpretation. In addition, the two sharp corners of the black object and the convex parts of the boundaries of both the white and black objects (all configurations with respect to the gray background) agree on depicting them in front of the gray background. The previous reasoning is summarized by the multi-scale features. The global integration mechanism can provide a consistent interpretation of the previous set of cues, assigning a non-uniform depth value to the pixels of the same object. However, if the bilateral filter is iterated until convergence, the final depth map will contain a constant depth value for each connected component. Eventually, the white object will be considered in front of the black one (not shown in Figure 7). The reason is that the white object is smaller and, hence, the averaging on the whole set will provide a higher value for the relative depth than the averaging on the larger set of black pixels. This means that, at the long run, the global integration mechanism favors small regions to be in front of larger ones in the case of inconsistency between local cues. In natural images, this interpretation can be biologically plausible and desirable from an evolutionary perspective [24]. Nevertheless, we have to be aware of this fact and consider it as a limitation of the integration mechanism proposed in the present invention. The other point to be noticed is that results provided by the algorithm depend on the number of iterations of the filter. For test images, at 100 iterations the algorithm may have not converged to a final solution but the interpretation in terms of relative depth is clear. The time of convergence mainly depends on the strength and location of the features and the size of the image.

Another example of the ability of the bilateral filter to integrate the local cues and provide a consistent depth map is shown in the fifth row of Figure 7, for a test image formed by four rectangular bars. In this case, a globally consistent depth relation cannot be established among some of the image objects (the bars). After some iterations of the filter we obtain a consistent interpretation of the local cues, establishing a depth gradient along each one of the bars. However, too many iterations of the filter (or its convergence) will lead to a relative depth map where each homogenous region will have a constant depth value. Depending on the spatial extent of the bilateral filter (the parameter $\sigma_s$ being or not comparable to the size of occlusion), each bar can be interpreted as a single object with the same relative depth for all its connected components, or broken into smaller pieces, where each component is treated as a separate object and may receive a different depth value.

Finally, the last two rows of Figure 7 present two symmetric sets separated by a staircase boundary and a sinusoidal boundary, respectively. Both configurations combine convex and concave cues along the boundary (corners and rounded shapes, respectively), generating mixed decisions for the depth relation of both sides. It can be seen that all features, independently of the scale, and consequently the multi-scale feature, provide similar cues for both sides of the boundary. In the case of the staircase, after some iterations, the relative depth map considers both sides at the same relative depth (the same constant depth value for the whole image). For the sinusoidal boundary, after the same number of iterations, one obtains a locally consistent interpretation that assigns a non homogenous depth to both sides of the boundary (convex regions of the boundary are above concave ones, similar to a zipper). As discussed above, this is a consequence of the convex cues being stronger than the corners to establish the relative depth between two sets. In any case, after some more iterations, the algorithm converges to the same solution as for the staircase boundary (not shown in Figure 7). A similar experiment was performed for a straight boundary but, as expected, no features were obtained at any of the scales (completely black images, not shown in the present invention).

Real images

We will compare the proposed approach with a set of four state-of-the-art monocular depth estimation techniques: two learning-based approaches, [27] and [53]; a figure/ground assignment technique [39]; and a Gestalt-based technique combining color and T-junction information to build a depth ordered image partition [45].

The original images belong to the Berkeley Segmentation Data Set [4], a well-known database formed by 500 natural images that is used for evaluation of image segmentation and edge detection techniques. In all results, the relative depth maps are encoded as gray level images with values in the interval [0, 1], where the brighter a pixel, the lower its relative depth (closer to the observer), and viceversa. To speed-up the computation of the relative depth maps for such a large set of images, we do not consider the original size of the image in the database, but a downsampled version by a factor of 2 (previously using proper low-pass filtering to prevent aliasing). In order to compute the multi-scale low-level depth features, the color image was downsampled again by a factor of 2 (that is, the original database image is downsampled by a factor of 4). Once the multi-scale features were extracted at this low resolution, they were upsampled using bilinear interpolation by a factor of 2 and the bilateral filter was applied, leading to relative depth maps with a half of the resolution of original dataset images.

For the proposed approach, all results have been computed using the multi-scale low-level features given by equation (21) with the scale dependent weights previously described. After normalizing the range of each image channel to the interval [0, 1], image bi-levels were extracted using a uniform quantization step $\Delta=1/8$ (i.e. a uniform quantization of the image range in 256 colors). Global integration was performed by the modified bilateral filter described before, using the parameters $\sigma_s = 5$ , $\sigma_c = 3/255$. The output depth maps are obtained after 100 iterations of the bilateral filter, which in general is close to convergence for the type and size of images used. For each image we show both, the multi-scale low-level features and the relative depth map. This way we can independently evaluate the quality of the features and the effect of the integration procedure.

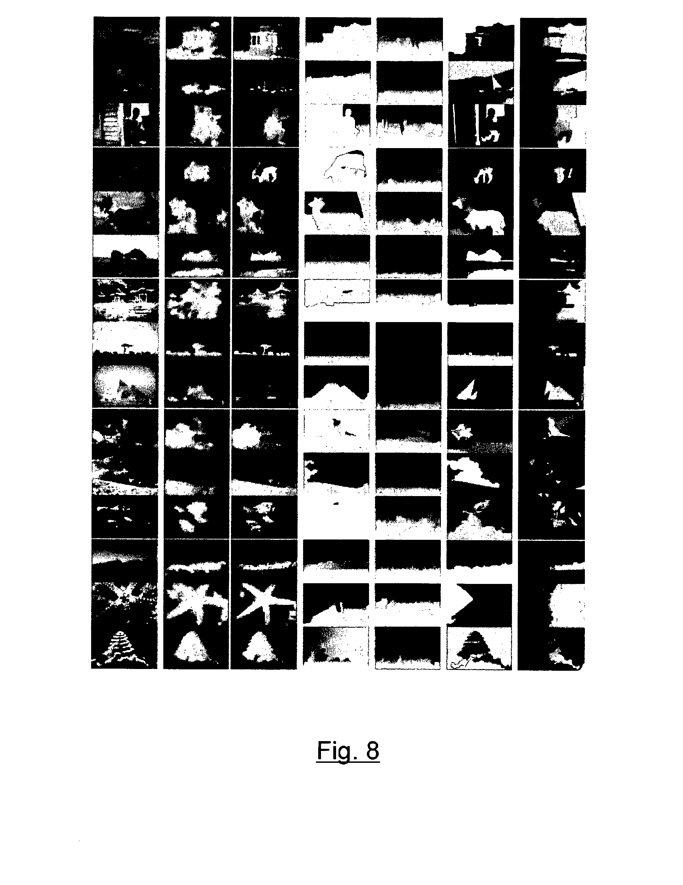

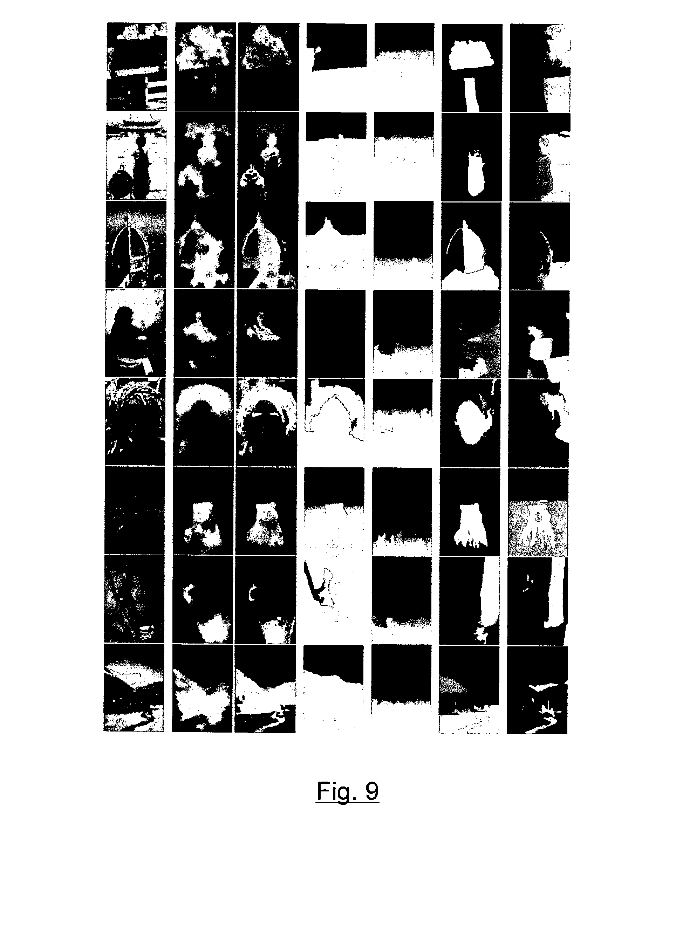

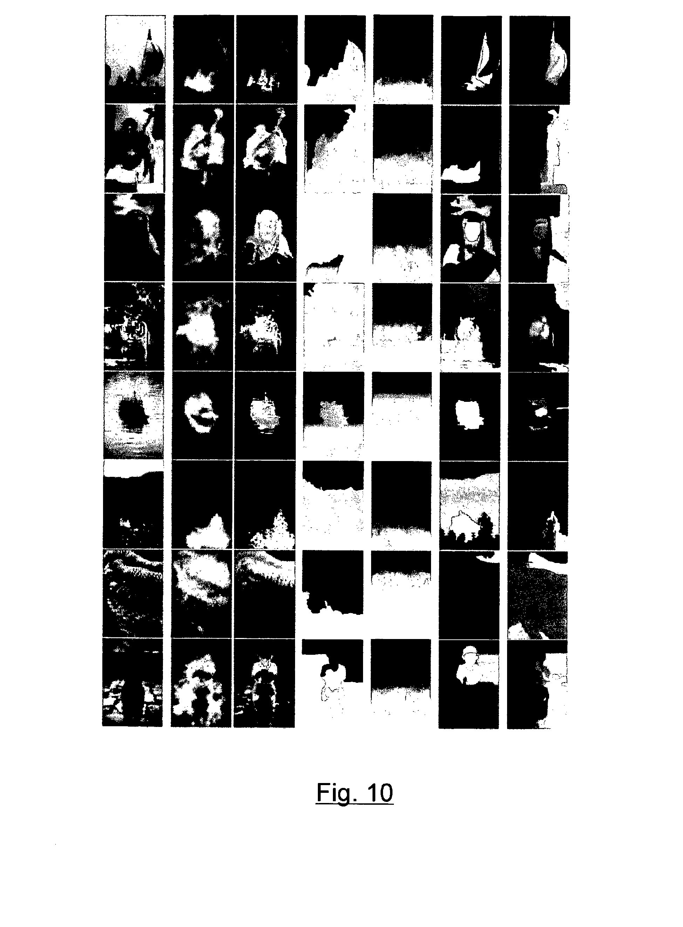

Some examples from the Berkeley Segmentation Data Set [4] are shown in **Figures 8, 9, 10** and **11.** For each row, columns from left to right:

- original image;
- multi-scale features;
- global integration after 100 iterations ($\sigma_s = 5$ , $\sigma_c = 3/255$);
- results by [27];
- results by [53];
- results by [45];
- results by [39].

First of all, we can observe that the multi-scale low-level features provide a rough estimate of the relative depth information (second column of the previous figures). Although the weights are computed in an automatic manner, the combination of the features at different scales provides a robust and stable descriptor that leads to features of similar resolution and characteristics for all images. In the third column of these figures, it can be seen how the global integration mechanism propagates the information provided by the low-level features according to the color information of the original image, leading to a fine relative depth map.

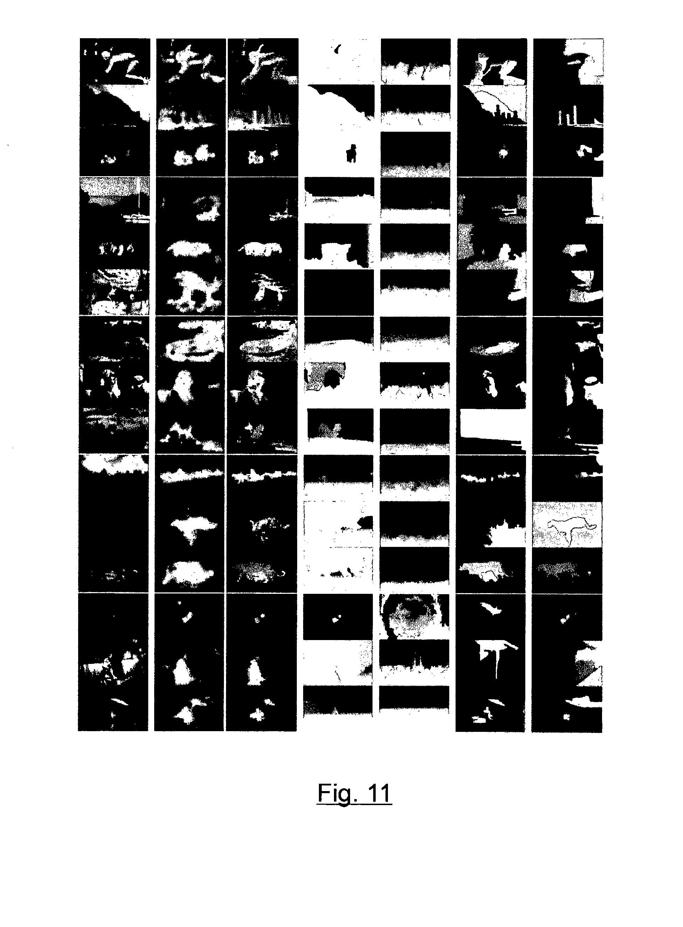

The fact that the provided depth map is fine is one of the most remarkable differences between the present approach and the state-of-the-art techniques. Those techniques are all segmentation-based. Particularly, [27] and [53] consider an over-segmented image partition as their input and, hence, their relative depth map is restricted to the initial partition; in turn, [39] and [45] provide as output result a relative depth segmentation (although in the case of [39] it would be possible to obtain a fine depth map skipping the last step of the method that averages the relative depth values of all pixels belonging to a certain region). Thus, they are sensitive to sub- and over-segmentation errors that can significantly condition the relative depth estimation. For instance, in Figures 8, 9, 10 and 11, it can be seen that, in general, the results provided by [27], [39], and [45] suffer from sub-segmentation errors. In contrast, the relative depth maps obtained by [53] are generally over-segmented and do not provide a clear depth gradient between different objects or components of the scene. The present approach does not involve image segmentation and, consequently, it is not conditioned by image partitioning. For instance, this allows providing smooth depth gradients in the relative depth map. Apart from [27], the other state-of-the-art techniques do not have this ability (constant depth values are assigned to all pixels belonging to the same region). Another consequence of the higher resolution of the fine relative depth maps provided by the present proposal is that the relative depth of thin structures is estimated with high accuracy. This is not the case for the segmentation-based techniques, which in most cases sub-segment this type of structures and fail to capture their relative depth. For instance, this situation can be seen in the fourth row of Figure 11.

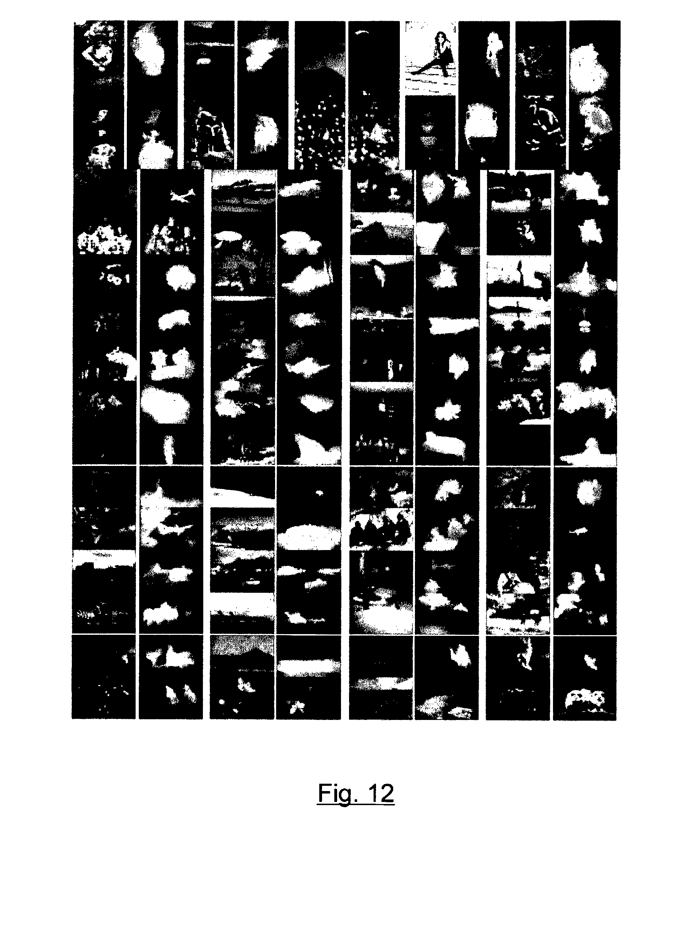

Considering the results for the 500 images of the Berkeley database [4], the relative depth maps provided by the proposed approach are in most cases correct and accurate. More examples of the relative depth maps computed by the present invention are shown in **Figure 12** (for each column, original image (left) and relative depth map extracted by the proposed approach (right), with the same parameter setting as in Figures 8, 9, 10 and 11), where it can be seen that the method succeeds to estimate depth maps of different types of images, some of them with a significant degree of complexity. The provided result outperforms the relative depth maps of the four state-of-the-art techniques.

The fine depth map obtained by the present method could provide an additional robust feature to the prior-art approaches, which can be also used to train the classifier in the case of [27] and [53], or to be part of the region similarity measure to create the image partition in [45].

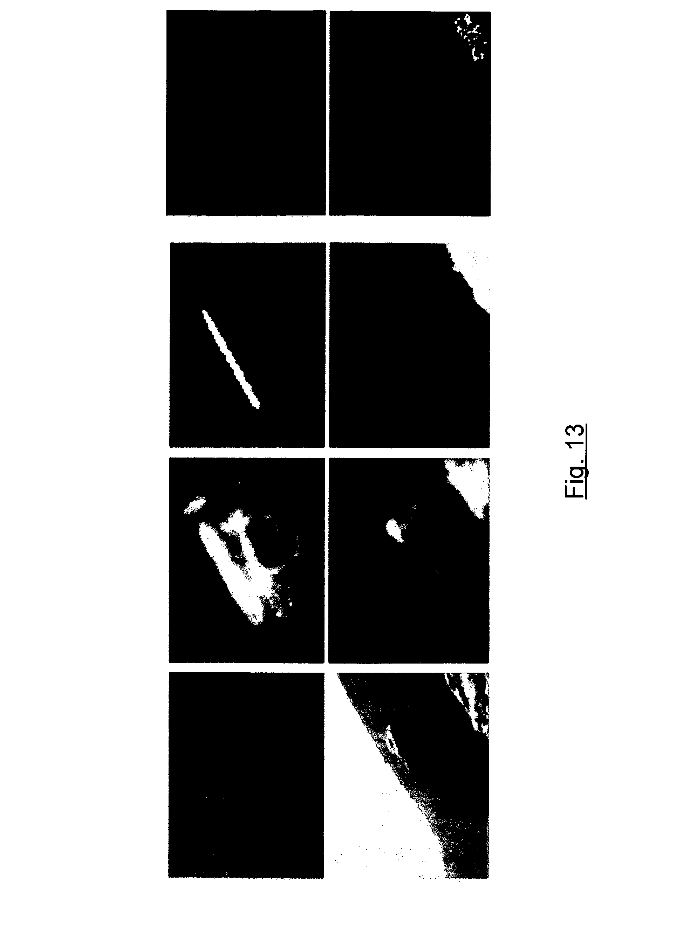

Finally, **Figure 13** presents a brief comparison with the technique proposed by [13]. As previously explained, this technique follows a similar approach to the present method in the sense that it computes local relative depth features that, in turn, are globally integrated using a neighborhood filter (a modified Yaroslavsky filter [63]). Nevertheless, the local features cannot be considered low-level, since they are the output of a T-junction estimation process that includes edge detection, edge completion, and detection of robust T-junction candidates. The method succeeds to estimate the relative depth maps of simple test images (including simple geometric objects, see an example in the first row of Figure 13) but fails in the case of natural images, such as landscapes. The second row of Figure 13 shows one example of failure (shown by the authors in [13]) and the result provided by the present approach.

**Claims**

1. Method for recovering a relative depth map from a single image or a sequence of still images, **characterized in**

**that** it comprises:

- decomposing the image into a plurality of sets $U_\lambda$, each one formed by the set of pixels whose intensity value is in a predefined intensity interval;

- decomposing each of the sets $U_\lambda$ into connected components $C_i^\lambda$, a connected component being defined as a subset of adjacent pixels according to a predefined pixel connectivity rule;

- for each connected component $C_i^\lambda$ of each set $U_\lambda$, computing membership measures indicative of the probability of each pixel $x \notin C_i^\lambda$ to belong to said connected component $C_i^\lambda$; each membership measure being a function of:

  • the distance $d(x, C_i^\lambda)$ from the pixel x to said connected component $C_i^\lambda$;

  • the curvature $\kappa(x, C_i^\lambda)$ of the level line of the distance function $d(x, C_i^\lambda)$ from the pixel $x$ to said connected component $C_i^\lambda$;

- for each pixel $x$ of the image, using the previous membership measures to compute a local depth feature $Z(x)$ indicative of the probability of the pixel $x$ belonging simultaneously to different connected components and which provides a relative depth order estimation at pixel $x$.

2. Method according to claim 1, further comprising generating, for each pixel $x$ of the image, a multi-scale local depth feature $Z^{MS}(x)$ by combining weighted averages of the local depth features $Z(x)$ at different scales ($s_0$, $s_1$,..., $s_N$) to make the relative depth order measures independent of the scale.

3. Method according to claim 2, further comprising applying a global integration process to the multi-scale local depth features $Z^{MS}(x)$ to obtain a consistent relative depth map.

4. Method according to claim 3, wherein the global integration process comprises a non-linear diffusion guided by color or gray level information of the pixels.

5. Method according to claim 4, wherein the non-linear diffusion is implemented by a modified bilateral filter that averages the multi-scale local depth features $Z^{MS}(x)$ in the neighborhood of a pixel x, weighted by the color or gray level similarity of the pixels.

6. Method according to any of claims 2 to 5, wherein for the generation of multi-scale local depth features $Z^{MS}(x)$ the weights $\omega(s_i)$ depending on the scale $s_i$ are selected according to the size distribution of connected components $C_i^\lambda$, giving more relevance to scales $s_i$ related to the size of the connected components with higher frequency of appearance on the image.

7. Method according to claim 6, further comprising computing the distribution of sizes of connected components $C_i^\lambda$ of the image to derive the weights $\omega(s_i)$.

8. Method according to any of claims 6 to 7, wherein the weights $\omega(s_i)$ have a decreasing behavior with increasing scales $s_i$.

9. Method according to claim 8, wherein the weights $\omega(s_i)$ are truncated or limited for lower scales $s_i$.

10. Method according to any of claims 6 to 9, wherein for the generation of multi-scale local depth features $Z^{MS}(x)$ the curvature $\kappa(x, C_i^\lambda)$ is computed after smoothing the distance function $d(x, C_i^\lambda)$ of the corresponding connected component $C_i^\lambda$ by a Gaussian kernel of standard deviation equal to the scale $s_i$ of analysis.

11. Method according to any of previous claims, wherein for the computation of a membership measure the method comprises computing a density of membership function $D(x, C_i^\lambda)$ indicative of the probability of the pixel $x$ belonging to a certain connected component $C_i^\lambda$, such that said function $D(x, C_i^\lambda)$ is increasing with the curvature $\kappa(x, C_i^\lambda)$ and decreasing with the distance $d(x, C_i^\lambda)$ from the pixel x to the connected component $C_i^\lambda$.

12. Method according to claim 11, wherein the density of membership function $D(x, C_i^\lambda)$ is such that:

$$D(x, C_i^\lambda) \propto e^{-E(x, C_i^\lambda)},$$

where $E(x, C_i^\lambda) = E_0(d(x, C_i^\lambda)) + E_1(\kappa(x, C_i^\lambda))$ is the energy of the pixel with respect to the connected component $C_i^\lambda$, such that $E_0(d(x, C_i^\lambda))$ is increasing in $d(x, C_i^\lambda)$ and $E_1(\kappa(x, C_i^\lambda))$ is decreasing in $\kappa(x, C_i^\lambda)$.

13. Method according to any of previous claims, wherein the local depth feature $Z(x)$ at a pixel x is obtained as the sum of the membership measures computed for said pixel $x$.

14. Method according to any of previous claims, wherein the intensity intervals are defined as [$\lambda -\Delta/2, \lambda +\Delta/2$), where $\Delta$ is a quantization step, and $\lambda \in \Delta Z$.

15. Method according to any of previous claims, wherein the pixel connectivity rule is defined according to any of the following rules:

- in still images the adjacent pixels are the top, bottom, left, and right pixel neighbors;
- in still images the adjacent pixels are the top, bottom, left, right, top-left, top-right, bottom-left, and bottom-right pixel neighbors;
- in a sequence of still images, adjacent pixels are defined similarly as for still images but taking into account the time dimension;
- in a sequence of still images, the spatial connectivity in the time dimension is defined in terms of the optical flow, defining as adjacent in time the closest pixel indicated by the direction of the motion vector.

**Patentansprüche**

1. Verfahren zum Wiederherstellen einer relativen Tiefenkarte anhand eines einzigen Bilds oder einer Sequenz von Standbildern, **dadurch gekennzeichnet, dass** es folgendes umfasst:

- Zerlegen des Bilds in eine Vielzahl an Gruppen $U_\lambda$, von denen jede durch die Pixelgruppe gebildet ist, deren Intensitätswert sich in einem vorbestimmten Intensitätsbereich befindet;

- Zerlegen jeder der Gruppen $U_\lambda$ in Zusammenhangskomponenten $C_i^\lambda$, wobei eine Zusammenhangskomponente als eine Untergruppe von benachbarten Pixeln nach einer vorbestimmten Pixelverbindungsregel definiert ist;

- Berechnen von Zugehörigkeitsmaßen für jede Zusammenhangskomponente $C_i^\lambda$ jeder Gruppe $U_\lambda$, welche die Wahrscheinlichkeit angibt, mit welcher jedes Pixel $\chi \notin C_i^\lambda$ zur genannten Zusammenhangskomponente $C_i^\lambda$ gehört, wobei jedes Zugehörigkeitsmaß eine Funktion ist von:

• dem Abstand $d(\chi, C_i^\lambda)$ vom Pixel $\chi$ zur genannten Zusammenhangskomponente $C_i^\lambda$;

• der Krümmung $\kappa(\chi, C_i^\lambda)$ der Höhenlinie der Abstandsfunktion $d(\chi, C_i^\lambda)$ vom Pixel $\chi$ zur genannten Zusammenhangskomponente $C_i^\lambda$;

- Verwenden der vorherigen Zugehörigkeitsmaße für jedes Pixel χ des Bilds zum Berechnen eines lokalen Tiefenmerkmals $Z(\chi)$, welches die Wahrscheinlichkeit angibt, mit welcher das Pixel χ gleichzeitig zu verschiedenen Zusammenhangskomponenten gehört, und welches eine Schätzung der Reihenfolge der relativen Tiefe an dem Pixel x bereitstellt.

2. Verfahren nach Anspruch 1, weiterhin umfassend das Erzeugen für jedes Pixel χ des Bilds eines lokalen Multiskala-Tiefenmerkmals $Z^{MS}(\chi)$ durch die Kombination der gewichteten Durchschnitte der lokalen Tiefenmerkmale $Z(\chi)$ bei verschiedenen Skalen ($s_0$, $s_1$,..., $s_N$), um die Maße der Reihenfolge der relativen Tiefe von der Skala unabhängig zu machen.

3. Verfahren nach Anspruch 2, weiterhin umfassend das Anwenden eines Gesamtintegrationsprozesses auf die lokalen Multiskala-Tiefenmerkmale $Z^{MS}(\chi)$ zum Erhalt einer konsistenten relativen Tiefenkarte.

4. Verfahren nach Anspruch 3, wobei der Gesamtintegrationsprozess eine nichtlineare Streuung umfasst, welche durch Farb- oder Graustufeninformationen der Pixel geführt ist.

5. Verfahren nach Anspruch 4, wobei die nichtlineare Streuung durch einen geänderten bilateralen Filter implementiert ist, welcher die lokalen Multi-Skala-Tiefenmerkmale $Z^{MS}(\chi)$, welche durch die Farb- oder Graustufenähnlichkeit der Pixel gewichtet sind, in der Nachbarschaft eines Pixels χ mittelt.

6. Verfahren nach einem der Ansprüche 2 bis 5, wobei zum Erzeugen von lokalen Multi-Skala-Tiefenmerkmalen $Z^{MS}(\chi)$ die Gewichtungen $\omega(s_i)$, welche von der Skala $s_i$ abhängen, nach der Größenverteilung von Zusammenhangskomponenten $C_i^\lambda$ ausgewählt werden, wobei Skalen $s_i$, welche mit der Größe der Zusammenhangskomponenten mit einer höheren Erscheinungshäufigkeit auf dem Bild verbunden sind, eine höhere Relevanz zugeteilt wird.

7. Verfahren nach Anspruch 6, weiterhin umfassend das Berechnen der Verteilung von Größen von Zusammenhangskomponenten $C_i^\lambda$ des Bilds zum Ableiten der Gewichtungen $\omega(s_i)$.

8. Verfahren nach einem der Ansprüche 6 bis 7, wobei die Gewichtungen $\omega(s_i)$ ein abnehmendes Verhalten mit zunehmenden Skalen $s_i$ haben.

9. Verfahren nach Anspruch 8, wobei die Gewichtungen $\omega(s_i)$ für niedrigere Skalen $s_i$ gekürzt bzw. begrenzt sind.

10. Verfahren nach einem der Ansprüche 6 bis 9, wobei zum Erzeugen von lokalen Multi-Skala-Tiefenmerkmalen $Z^{MS}(\chi)$ die Krümmung $\kappa(\chi, C_i^\lambda)$ nach dem Glätten der Abstandsfunktion $d(\chi, C_i^\lambda)$ der entsprechenden Zusammenhangskomponente $C_i^\lambda$ durch einen Gaußschen Kernel einer Standardabweichung, entsprechend der Analyse-Skala $s_i$, berechnet wird.

11. Verfahren nach einem der vorherigen Ansprüche, wobei das Verfahren zur Berechnung eines Zugehörigkeitsmaßes das Berechnen einer Dichte der Zugehörigkeitsfunktion $D(\chi, C_i^\lambda)$ umfasst, welche die Wahrscheinlichkeit angibt, dass das Pixel χ zu einer bestimmten Zusammenhangskomponente $C_i^\lambda$ gehört, so dass sich die genannte Funktion $D(\chi, C_i^\lambda)$ mit der Krümmung $\kappa(\chi, C_i^\lambda)$ erhöht und mit dem Abstand $d(\chi, C_i^\lambda)$ vom Pixel χ zur Zusammenhangskomponente $C_i^\lambda$ verringert.

12. Verfahren nach Anspruch 11, wobei die Dichte der Zugehörigkeitsfunktion $D(\chi, C_i^\lambda)$ derart ist, dass gilt:

$$D(x, C_i^\lambda) \propto e^{-E(x, C_i^\lambda)},$$

wobei $E(\chi, C_i^\lambda) = E_0(d(\chi, C_i^\lambda)) + E_1(\kappa(\chi, C_i^\lambda))$ die Energie des Pixels bezüglich der Zusammenhangskomponente $C_i^\lambda$ ist, so dass sich $E_0(d(\chi, C_i^\lambda))$ um $d(\chi, C_i^\lambda)$ erhöht und $E_1(\kappa(\chi, C_i^\lambda))$ um $\kappa(\chi, C_i^\lambda)$ verringert.

**13.** Verfahren nach einem der vorherigen Ansprüche, wobei das lokale Tiefenmerkmal $Z(x)$ bei einem Pixel $\chi$ als Summe der Zugehörigkeitsmaße, welche für das genannte Pixel $\chi$ berechnet wird, erhalten wird.

**14.** Verfahren nach einem der vorherigen Ansprüche, wobei die Intensitätsbereiche als ($\lambda - \Delta/2, \lambda + \Delta/2$) definiert werden, wobei $\Delta$ ein Quantifizierungsschritt ist und $\lambda \in \Delta Z$ gilt.

**15.** Verfahren nach einem der vorherigen Ansprüche, wobei die Pixelverbindungsregel nach einer der folgenden Regeln definiert ist:

- bei Standbildern sind die benachbarten Pixel die oberen, unteren, linken und rechten Pixelnachbarn;
- bei Standbildern sind die benachbarten Pixel die oberen, unteren, linken, rechten, oberen linken, oberen rechten, unteren linken und unteren rechten Pixelnachbarn;
- in einer Sequenz von Standbildern sind benachbarte Pixel ähnlich definiert wie für Standbilder, aber unter Berücksichtigung der zeitlichen Dimension;
- in einer Sequenz von Standbildern ist die räumliche Verbindung in der zeitlichen Dimension bezüglich des optischen Flusses definiert, wobei als zeitlich benachbart das nächste Pixel definiert wird, das durch die Richtung des Bewegungsvektors angegeben ist.

**Revendications**

**1.** Procédé pour récupérer une carte de profondeur relative à partir d'une image unique ou d'une séquence d'images fixes, **caractérisé en ce qu'**il comprend :

- la décomposition de l'image en une pluralité d'ensembles $U_\lambda$, chacun formé par l'ensemble de pixels dont la valeur d'intensité est dans un intervalle d'intensité prédéfini ;

- la décomposition de chacun des ensembles $U_\lambda$ en composants connectés $C_i^\lambda$, un composant connecté étant défini comme un sous-ensemble de pixels adjacents selon une règle de connectivité de pixel prédéfinie ;

- pour chaque composant connecté $C_i^\lambda$ de chaque ensemble $U_\lambda$, le calcul des mesures d'adhésion indiquant la probabilité de chaque pixel $x \notin C_i^\lambda$ d'appartenir audit composant connecté $C_i^\lambda$ ;

chaque mesure d'adhésion étant une fonction de :

• la distance $d(x, C_i^\lambda)$ entre le pixel x et ledit composant connecté $C_i^\lambda$ ;

• la courbure $k(x, C_i^\lambda)$ de la ligne de niveau de la fonction de distance $d(x, C_i^\lambda)$ entre le pixel x et ledit composant connecté $C_i^\lambda$ ;

- pour chaque pixel x de l'image, l'utilisation des mesures d'adhésion précédentes pour calculer une caractéristique de profondeur locale $Z(x)$ indiquant la probabilité du pixel x d'appartenir simultanément à différents composants connectés et qui fournit une estimation de l'ordre de profondeur relative au pixel *x*.

**2.** Procédé selon la revendication 1, comprenant en outre la génération, pour chaque pixel *x* de l'image, d'une caractéristique de profondeur locale à échelle multiple $Z^{MS}(x)$ en combinant des moyennes pondérées des caractéristiques de profondeur locale $Z(x)$ à différentes échelles ($s_0, s_1, ..., s_N$) pour réaliser les mesures de l'ordre de profondeur relative indépendamment de l'échelle.

**3.** Procédé selon la revendication 2, comprenant en outre l'application d'un processus d'intégration globale aux caractéristiques de profondeur locale à échelle multiple $Z^{MS}(x)$ pour obtenir une carte de profondeur relative consistante.

4. Procédé selon la revendication 3, dans lequel le processus d'intégration globale comprend une diffusion non-linéaire guidée par des informations de la couleur ou du niveau de gris des pixels.

5. Procédé selon la revendication 4, dans lequel la diffusion non-linéaire est mise en oeuvre par un filtre bilatéral modifié qui fait la moyenne des caractéristiques de profondeur locale à échelle multiple $Z^{MS}(x)$ dans le voisinage d'un pixel x, pondéré par la similarité de la couleur ou du niveau de gris des pixels.

6. Procédé selon l'une quelconque des revendications 2 à 5, dans lequel pour la génération de caractéristiques de profondeur locale à échelle multiple $Z^{MS}(x)$ les pondérations $\omega(s_i)$ en fonction de l'échelle $s_i$ sont choisies selon la distribution de la taille des composants connectés $C_i^\lambda$, donnant plus d'importance aux échelles $s_i$ en rapport avec la taille des composants connectés avec une plus haute fréquence d'apparence sur l'image.

7. Procédé selon la revendication 6, comprenant en outre le calcul de la distribution des tailles des composants connectés $C_i^\lambda$ de l'image pour en tirer les pondérations $\omega(s_i)$.

8. Procédé selon l'une quelconque des revendications 6 à 7, dans lequel les pondérations $\omega(s_i)$ ont un comportement décroissant avec des échelles croissantes $s_i$.

9. Procédé selon la revendication 8, dans lequel les pondérations $\omega(s_i)$ sont tronquées ou limitées pour des échelles plus petites $s_i$.

10. Procédé selon l'une quelconque des revendications 6 à 9, dans lequel pour la génération de caractéristiques de profondeur locale à échelle multiple $Z^{MS}(x)$ la courbure $k(x, C_i^\lambda)$ est calculée après le lissage de la fonction de distance $d(x, C_i^\lambda$ du composant connecté correspondant $C_i^\lambda$ par un noyau Gaussien de déviation standard égale à l'échelle $s_i$ d'analyse.

11. Procédé selon l'une quelconque des revendications précédentes, dans lequel pour le calcul d'une mesure d'adhésion le procédé comprend calculer une densité de fonction d'adhésion $D(x, C_i^\lambda)$ indiquant la probabilité du pixel x d'appartenir à un certain composant connecté $C_i^\lambda$, de sorte que ladite fonction $D(x, C_i^\lambda)$ croît avec la courbure $k(x, C_i^\lambda)$ et décroît avec la distance $d(x, C_i^\lambda)$ entre le pixel x et le composant connecté $C_i^\lambda$.

12. Procédé selon la revendication 11, dans lequel la densité de fonction d'adhésion $\mathrm{D}(x, C_i^\lambda)$ est telle que :

$$D(x, C_i^\lambda) \propto e^{-E(x, C_i^\lambda)},$$

où $E(x, C_i^\lambda) = E_0(d(x, C_i^\lambda)) + E_1(k(x, C_i^\lambda))$ est l'énergie du pixel par rapport au composant connecté $C_i^\lambda$, de sorte que $E_0(d(x, C_i^\lambda))$ croît dans $d(x, C_i^\lambda)$ et $E_1(k(x, C_i^\lambda))$ décroît dans $k(x, C_i^\lambda)$.

13. Procédé selon l'une quelconque des revendications précédentes, dans lequel la caractéristique de profondeur locale $Z(x)$ à un pixel x est obtenue comme la somme des mesures d'adhésion calculées pour ledit pixel x.

14. Procédé selon l'une quelconque des revendications précédentes, dans lequel les intervalles d'intensité sont définis comme $[\lambda - \Delta/2, \lambda + \Delta/2)$, où $\Delta$ est une étape de quantification, et $\lambda \in \Delta Z$.

15. Procédé selon l'une quelconque des revendications précédentes, dans lequel la règle de connectivité de pixel est définie selon l'une quelconque des règles suivantes :

- dans des images fixes les pixels adjacents sont les pixels voisins du haut, du bas, de gauche, et de droite ;
- dans des images fixes les pixels adjacents sont les pixels voisins du haut, du bas, de gauche, de droite, d'en haut à gauche, d'en haut à droite, d'en bas à gauche, et d'en bas à droite ;

- dans une séquence d'images fixes, les pixels adjacents sont similairement définis comme pour des images fixes mais en tenant compte de la dimension temporelle ;

- dans une séquence d'images fixes, la connectivité spatiale dans la dimension temporelle est définie en termes du flux optique, définissant comme adjacent en temps le pixel le plus proche indiqué par la direction du vecteur de déplacement.

Fig. 1

Fig. 2

Fig. 3

Fig. 4

Fig. 5

Fig. 6

Fig. 7

Fig. 8

Fig. 9

Fig. 10

Fig. 11

Fig. 12

Fig. 13

**EP 2 747 028 B1**

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2011138472 A1
- WO 2008016885 A2
- US 20100079448 A
- WO 2006003577 A1
- EP 2169619 A2
- US 2010079453 A1
- WO 2005052858 A1
- WO 2005015497 A1
- US 20110188773 A1
- US 20120002871 A1

- US 20120293499 A1
- US 2006061569 A1
- US 20080247670 A1
- US 20070024614 A1
- US 2010310176 A1
- US 2010080481 A1
- CN 102509294 A
- WO 10018880 A1
- US 20070146232 A1

**Non-patent literature cited in the description**

- **ALVAREZ, L. ; GOUSSEAU, Y. ; MOREL, J.** Scales in natural images and a consequence on their bounded variation norm. *Scale-Space Theories in Computer Vision,* 1999, 247-258
- **ALVAREZ, L. ; GOUSSEAU, Y. ; MOREL, J.** The size of objects in natural and artificial images. *Advances in Imaging and Electron Physics,* 1999, vol. 111, 167-242
- **AMER, M. ; RAICH, R. ; TODOROVIC, S.** Monocular extraction of 2.1 d sketch. *Proc. of the International Conference on Image Processing,* 2010
- **ARBELAEZ, P. ; MAIRE, M. ; FOWLKES, C. ; MALIK, J.** Contour detection and hierarchical image segmentation. *IEEE Trans. Pattern Anal. Mach. Intell.,* 2011, vol. 33 (5), 898-916
- **BORDENAVE, C. ; GOUSSEAU, Y ; ROUE , F.** The dead leaves model: a general tessellation modeling occlusion. *Advances in Applied Probability,* 2006, vol. 38 (1), 31-46
- A non-local algorithm for image denoising. **BUADES, A. ; COLL, B. ; MOREL, J.** Computer Vision and Pattern Recognition, 2005. CVPR 2005. IEEE Computer Society Conference. Ieee, 2005, vol. 2, 60-65
- **BUADES, A. ; LE, T. ; MOREL, J. ; VESE, L.** Fast cartoon+ texture image filters. *Image Processing, IEEE Transactions,* 2010, vol. 19 (8), 1978-1986
- **CALDERERO, F. ; MARQUES, F.** Region merging techniques using information theory statistical measures. *Image Processing, IEEE Transactions,* 2010, vol. 19 (6), 1567-1586
- **CASELLES, V. ; COLL, B. ; MOREL, J.** A kanizsa programme. *ICAOS'96,* 1996, 356-359

- **CASELLES, V. ; COLL, B. ; MOREL, J.** Topographic maps and local contrast changes in natural images. *International Journal of Computer Vision,* 1999, vol. 33 (1), 5
- **CASELLES, V. ; MONASSE, P.** Geometric description of images as topographic maps. Springer-Verlag New York Inc, 2010, vol. 1984
- **DARRELL, T. ; PENTLAND, A.** Cooperative robust estimation using layers of support. Pattern Analysis and Machine Intelligence. *IEEE Transactions,* 1995, vol. 17 (5), 474-487
- **DIMICCOLI, M. ; MOREL, J. ; SALEMBIER, P.** Monocular depth by nonlinear di usion. *Computer Vision, Graphics & Image Processing, 2008. ICVGIP'08. Sixth Indian Conference,* 2008, 95-102
- Exploiting t-junctions for depth segregation in single images. **DIMICCOLI, M. ; SALEMBIER, P.** Acoustics, Speech and Signal Processing, 2009. ICASSP 2009. IEEE International Conference. IEEE, 2009, 1229-1232
- Hierarchical region-based representation for segmentation and filtering with depth in single images. **DIMICCOLI, M. ; SALEMBIER, P.** Image Processing (ICIP), 2009 16th IEEE International Conference. IEEE, 2009, 3533-3536
- Flash photography enhancement via intrinsic relighting. **EISEMANN, E. ; DURAND, F.** ACM Transactions on Graphics (TOG). ACM, 2004, vol. 23, 673-678
- **FAVARO, P. ; SOATTO, S. ; BURGER, M. ; OSHER, S.** Shape from defocus via di usion. Pattern Analysis and Machine Intelligence. *IEEE Transactions,* 2008, vol. 30 (3), 518-531

41

- **FELDMAN, D. ; WEINSHALL, D.** Motion segmentation and depth ordering using an occlusion detector. Pattern Analysis and Machine Intelligence. *IEEE Transactions,* 2008, vol. 30 (7), 1171-1185
- **FOWLKES, C. ; MARTIN, D. ; MALIK, J.** Local figure-ground cues are valid for natural images. *Journal of Vision,* 2007, vol. 7 (8
- **FROYEN, V. ; FELDMAN, J. ; SINGH, M.** A bayesian framework for figure-ground interpretation. *Advances in Neural Information Processing Systems,* 2010, 23
- **FROYEN, V. ; FELDMAN, J. ; SINGH, M.** Local propagation of border-ownership. *Journal of Vision,* 2010, vol. 10 (7), 1176-1176
- **FROYEN, V. ; KOGO, N. ; FELDMAN, J. ; SINGH, M. ; WAGEMANS, J.** Integration of contour and skeleton based cues in the reconstruction of surface structure. *Perception,* 2011, vol. 40, 175a
- Bayesian inference for layer representation with mixed markov random field. **GAO, R. ; WU, T. ; ZHU, S. ; SANG, N.** Energy Minimization Methods in Computer Vision and Pattern Recognition. Springer, 2007, 213-224
- **GIBSON, J.** *The ecological approach to visual perception. Lawrence Erlbaum,* 1986
- **GOLDSTEIN, E.B.** Sensation and perception. 2002
- **GOUSSEAU, Y. ; MOREL, J.** Are natural images of bounded variation?. *SIAM journal on mathematical analysis,* 2001, vol. 33 (3), 634-648
- **HOIEM, D. ; EFROS, A. ; HEBERT, M.** Recovering occlusion boundaries from an image. *International Journal of Computer Vision,* 2011, vol. 91 (3), 328-346
- **HOWARD, I.** erceiving in Depth, Volume 3: Other Mechanisms of Depth Perception. Oxford Univ Pr, 2012, vol. 29
- **KANIZSA, G.** *Grammatica del vedere: saggi su percezione e gestalt, ii mulino,* 1980
- **KIM, S. ; FELDMAN, J.** Globally inconsistent figure/ground relations induced by a negative part. *Journal of Vision,* 2009, vol. 9 (10
- **KOGO, N. ; FROYEN, V. ; FELDMAN, J. ; SINGH, M. ; WAGEMANS, J.** Integration of local and global cues to reconstruct surface structure. *Journal of Vision,* 2011, vol. 11 (11
- **KOGO, N. ; GALLI, A. ; WAGEMANS, J.** Switching dynamics of border ownership: A stochastic model for bi-stable perception. *Vision Research,* 2011, vol. 51, 2085-2098
- **KOGO, N. ; STRECHA, C. ; VAN GOOL, L. ; WAGEMANS, J.** Surface construction by a 2-d differentiation-integration process: A neurocomputational model for perceived border ownership, depth, and lightness in kanizsa figures. *Psychological review,* 2010, vol. 117 (2), 406
- **KOPF, J. ; COHEN, M. ; LISCHINSKI, D. ; UYTTEN-DAELE, M.** Joint bilateral upsampling. *ACM Transactions on Graphics,* 2007, vol. 26 (3), 96
- **LEE, S. ; SHARMA, S.** Real-time disparity estimation algorithm for stereo camera systems. *Consumer Electronics, IEEE Transactions,* 2011, vol. 57 (3), 1018
- Boundary ownership by lifting to 2.1 d. **LEICHTER, I. ; LINDENBAUM, M.** Computer Vision, 2009 IEEE 12th International Conference. IEEE, 2009, 9-16
- **LINDEBERG, T.** Scale-space theory in computer vision. Springer, 1994
- Single image depth estimation from predicted semantic labels. **LIU, B. ; GOULD, S. ; KOLLER, D.** Computer Vision and Pattern Recognition (CVPR), 2010 IEEE Conference. IEEE, 2010, 1253-1260
- **MAIRE, M.** Simultaneous segmentation and figure/ground organization using angular embedding. *Computer Vision{ECCV 2010,* 2010, 450-464
- **MARR, D.** *Vision: A computational approach,* 1982
- **METZGER, W.** Gesetze des sehens (die lehre vom sehen der formen und dinge des raumes und der bewegung). *Frankfurt/M.: Kramer,* 1975
- Recovery of relative depth from a single observation using an uncalibrated (real-aperture) camera. **NAMBOODIRI, V. ; CHAUDHURI, S.** Computer Vision and Pattern Recognition, 2008. CVPR 2008. IEEE Conference. IEEE, 2008, 1-6
- The 2.1-d sketch. **NITZBERG, M. ; MUMFORD, D.** Computer Vision, 1990. Proceedings, Third International Conference. IEEE, 1990, 138-144
- **NITZBERG, M. ; MUMFORD, D. ; SHIOTA, T.** Filtering, segmentation, and depth. Springer, 1993, vol. 662
- Occlusion-based depth ordering on monocular images with binary partition tree. **PALOU, G. ; SALEMBIER, P.** Acoustics, Speech and Signal Processing (ICASSP), 2011 IEEE International Conference. IEEE, 2011, 1093-1096
- **PARIDA, L. ; GEIGER, D. ; HUMMEL, R.** Junctions: Detection, classification, and reconstruction. Pattern Analysis and Machine Intelligence. *IEEE Transactions,* 1998, vol. 20 (7), 687-698
- **PARIS, S. ; DURAND, F.** A fast approximation of the bilateral filter using a signal processing approach. *International journal of computer vision,* 2009, vol. 81 (1), 24-52
- **PETERSON, M. ; SKOW, E.** Inhibitory competition between shape properties in figure-ground perception. *Journal of Experimental Psychology: Human Perception and Performance,* 2008, vol. 34 (2), 251

- Digital photography with flash and no-flash image pairs. **PETSCHNIGG, G. ; SZELISKI, R. ; AGRAWALA, M. ; COHEN, M. ; HOPPE, H. ; TOYA-MA, K.** ACM Transactions on Graphics (TOG). ACM, 2004, vol. 23, 664
- Separable bilateral filtering for fast video preprocessing. **PHAM, T. ; VAN VLIET, L.** Multimedia and Expo, 2005. ICME 2005. IEEE International Conference. IEEE, 2005, 4
- **RENSINK, R. ; ENNS, J.** Early completion of occluded objects. *Vision research,* 1998, vol. 38 (15-16), 2489-2505
- **RUBIN, N.** Figure and ground in the brain. *Nature Neuroscience,* 2001, vol. 4, 857-858
- **SAXENA, A. ; CHUNG, S. ; NG, A.** 3-d depth reconstruction from a single still image. *International Journal of Computer Vision,* 2008, vol. 76 (1), 53-69
- **SERRA, J.** Image analysis and mathematical morphology. Academic Press, 1982, vol. 1
- **SOILLE, P.** Morphological image analysis: principles and applications. Springer-Verlag New York, Inc, 2003
- Bilateral filtering for gray and color images. **TOMASI, C. ; MANDUCHI, R.** Computer Vision, 1998. Sixth International Conference. IEEE, 1998, 839-846
- **TORRALBA, A. ; OLIVA, A.** Depth estimation from image structure. *Pattern Analysis and Machine Intelligence, IEEE Transactions,* 2002, vol. 24 (9), 1226-1238
- **VINCENT, L. ; SOILLE, P.** Watersheds in digital spaces: an efficient algorithm based on immersion simulations. *IEEE transactions on pattern analysis and machine intelligence,* 1991, vol. 13 (6), 583-598
- **VON GIOI, R. ; JAKUBOWICZ, J. ; MOREL, J. ; RANDALL, G.** Lsd: A fast line segment detector with a false detection control. *Pattern Analysis and Machine Intelligence, IEEE Transactions,* 2010, vol. 32 (4), 722-732
- **WANG, J. ; ADELSON, E.** Representing moving images with layers. *Image Processing, IEEE Transactions,* 1994, vol. 3 (5), 625-638
- **WILLIAMS, L. ; JACOBS, D.** Stochastic completion fields: A neural model of illusory contour shape and salience. *Neural Computation; Neural Computation,* 1997
- Spatial-depth super resolution for range images. **YANG, Q. ; YANG, R. ; DAVIS, J. ; NISTÉR, D.** Computer Vision and Pattern Recognition, 2007. CVPR'07. IEEE Conference. IEEE, 2007, 1-8
- **YAROSLAVSKY, L.** Digital picture processing. An introduction. Springer-Verlag, 1985, vol. 1
- Angular embedding: from jarring intensity differences to perceived luminance. **YU, S.** Computer Vision and Pattern Recognition, 2009. CVPR 2009. IEEE Conference. IEEE, 2009, 2302-2309
- **ZHOU, H. ; FRIEDMAN, H. ; VON DER HEYDT, R.** Coding of border ownership in monkey visual cortex. *The Journal of Neuroscience,* 2000, vol. 20 (17), 6594-6611